(19) Europäisches Patentamt
European Patent Office
Office européen des brevets

(11) EP 4 458 850 A1

(12) **EUROPEAN PATENT APPLICATION**
published in accordance with Art. 153(4) EPC

(43) Date of publication:
**06.11.2024 Bulletin 2024/45**

(21) Application number: **22914871.3**

(22) Date of filing: **28.12.2022**

(51) International Patent Classification (IPC):
***C07K 16/28*** (2006.01) ***C12N 15/13*** (2006.01)
***A61P 35/00*** (2006.01)

(52) Cooperative Patent Classification (CPC):
**A61P 35/00; C07K 16/00; C07K 16/28**

(86) International application number:
**PCT/CN2022/142644**

(87) International publication number:
**WO 2023/125619 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2021 CN 202111623091**
**10.03.2022 CN 202210230033**

(71) Applicants:
- **Jiangsu Hengrui Pharmaceuticals Co., Ltd.**
  **Lianyungang, Jiangsu 222047 (CN)**
- **Shanghai Hengrui Pharmaceutical Co., Ltd.**
  **Shanghai 200245 (CN)**

(72) Inventors:
- **YANG, Yang**
  **Shanghai 200245 (CN)**
- **YANG, Chunpeng**
  **Shanghai 200245 (CN)**
- **LIN, Guang**
  **Shanghai 200245 (CN)**
- **JIN, Xinsheng**
  **Shanghai 200245 (CN)**
- **TAO, Weikang**
  **Shanghai 200245 (CN)**

(74) Representative: **Regimbeau**
**20, rue de Chazelles**
**75847 Paris Cedex 17 (FR)**

Remarks:
The complete document including Reference Table(s) and the Sequence Listing(s) can be downloaded from the EPO website

(54) **ANTI-ROR1 ANTIBODY, AND ANTI-ROR1 ANTIBODY-DRUG CONJUGATE AND MEDICAL USES THEREOF**

(57) An anti-ROR1 antibody, and an anti-ROR1 antibody-drug conjugate and the medical uses thereof.

EP 4 458 850 A1

## Description

The present application claims priority to the Chinese Patent Application (Application No. CN202111623091.0) filed on Dec. 28, 2021 and the Chinese Patent Application (Application No. CN202210230033.X) filed on Mar. 10, 2022.

## TECHNICAL FIELD

The present disclosure relates to an anti-ROR1 antibody and an anti-ROR1 immunoconjugate, a preparation method therefor, a pharmaceutical composition comprising same, and use thereof in the preparation of a drug for treating a ROR1-mediated disease or disorder, particularly in the preparation of an anti-cancer drug.

## BACKGROUND

The statements herein merely provide background information related to the present disclosure and may not necessarily constitute the prior art.

Receptor tyrosine kinase-like orphan receptor 1 (ROR1) belongs to the family of receptor tyrosine kinases (RTKs). ROR1 is a typical type I single-pass transmembrane protein on the cell membrane and contains three different domains in the extracellular region, i.e., an immunoglobulin-like (Ig-like) domain, a cysteine-rich frizzled domain (FZD), and a kringle domain (KD). The intracellular region is composed of a tyrosine kinase domain, which is mainly involved in downstream RTK signaling. As a RTK protein, intracytoplasmic activation of ROR1 requires two steps: 1. an increase in intrinsic catalytic activity; and 2. formation of binding sites for downstream signaling pathway proteins. These two steps are accomplished by autophosphorylation of intracellular tyrosine residues following ligand-mediated oligomerization. Current studies show that ROR1 can be expressed in the skeletal, respiratory, and cardiac systems and in the neuronal synaptic cells of central neurons during different stages of embryonic development, and its expression is markedly down-regulated after birth. ROR1 can bind to WNT5a protein and activate noncanonical WNT signaling pathways in tumor cells, maintain stem cell properties of tumor cells, including proliferation, migration and differentiation, and is a surface marker of cancer stem cells (CSCs). The correlation of ROR1 expression with tumors was first determined in B-cell chronic lymphocytic leukemia (CLL). Studies showed that ROR1 is highly expressed in various hematological tumors, including mantle cell lymphoma (MCL), marginal zone lymphoma (MZL), diffuse large B-cell lymphoma (DLBCL), follicular lymphoma (FL), etc. Further studies on ROR1 expression level were extended to solid tumors. Studies showed that a series of solid tumors such as triple-negative breast cancer (TNBC), different types of lung cancer, different types of ovarian cancer, pancreatic cancer, and the like all exhibit different degrees of ROR1 expression.

ROR1 expression is not detected in most normal tissues, including important human organs such as heart, spleen, lung, and liver, and certain expression is only detected in parathyroid, pancreatic islet, and gastrointestinal tissues. ROR1 can be an ideal target for ADC molecule development given the large difference in the expression between normal tissues and tumor tissues, and different degrees of expression in various tumors, including hematological tumors and solid tumors.

## SUMMARY

The present disclosure relates to an isolated anti-ROR1 antibody, comprising:

a HCDR1, a HCDR2, and a HCDR3 comprised in a heavy chain variable region set forth in SEQ ID NO: 2; and
a LCDR1, a LCDR2, and a LCDR3 comprised in a light chain variable region set forth in SEQ ID NO: 3.

In some embodiments, the anti-ROR1 antibody described above comprises a heavy chain variable region and a light chain variable region, wherein:

the heavy chain variable region comprises a HCDR1 set forth in SEQ ID NO: 4, 16 or 22, a HCDR2 set forth in SEQ ID NO: 5, 17 or 23, and a HCDR3 set forth in SEQ ID NO: 6, 18 or 24, respectively;
the light chain variable region comprises a LCDR1 set forth in SEQ ID NO: 7, 19 or 25, a LCDR2 set forth in SEQ ID NO: 8, 20 or 26, and a LCDR3 set forth in SEQ ID NO: 9, 21 or 27, respectively;
the CDR regions described above are defined according to the Kabat, IMGT, or Chothia numbering scheme.

In some embodiments, the anti-ROR1 antibody described above comprises a heavy chain variable region and a light chain variable region, wherein:

the heavy chain variable region comprises a HCDR1, a HCDR2, and a HCDR3 set forth in SEQ ID NO: 4, SEQ ID

NO: 5, and SEQ ID NO: 6, respectively;
the light chain variable region comprises a LCDR1, a LCDR2, and a LCDR3 set forth in SEQ ID NO: 7, SEQ ID NO: 8, and SEQ ID NO: 9, respectively;
the CDR regions described above are defined according to the Kabat numbering scheme.

In some embodiments, provided is the anti-ROR1 antibody according to any one of the above, wherein the anti-ROR1 antibody is a full-length human antibody or an antigen-binding fragment thereof.
In some embodiments, provided is the anti-ROR1 antibody according to any one of the above, which comprises a heavy chain variable region and a light chain variable region, wherein:

the heavy chain variable region comprises an amino acid sequence having at least 90%, 91%, 92%, 93%, 94%, 95%, 96%, 97%, 98%, 99%, or 100% sequence identity to SEQ ID NO: 2, and/or
the light chain variable region comprises an amino acid sequence having at least 90%, 91%, 92%, 93%, 94%, 95%, 96%, 97%, 98%, 99%, or 100% sequence identity to SEQ ID NO: 3.

In some embodiments, provided is the anti-ROR1 antibody according to any one of the above, which comprises a heavy chain variable region and a light chain variable region, wherein:

the amino acid sequence of the heavy chain variable region is set forth in SEQ ID NO: 2, and
the amino acid sequence of the light chain variable region is set forth in SEQ ID NO: 3. In some embodiments, provided is the anti-ROR1 antibody according to any one of the above, wherein the antibody further comprises an antibody heavy chain constant region and a light chain constant region. Preferably, the heavy chain constant region is selected from the group consisting of human IgG1, IgG2, IgG3, and IgG4 constant regions, and the light chain constant region is selected from the group consisting of human antibody $\kappa$ and $\lambda$ chain constant regions. More preferably, the antibody comprises a heavy chain constant region set forth in SEQ ID NO: 10 and a light chain constant region set forth in SEQ ID NO: 11.

In some embodiments, provided is the anti-ROR1 antibody according to any one of the above, which comprises:

a heavy chain having at least 85%, 90%, 91%, 92%, 93%, 94%, 95%, 96%, 97%, 98%, 99%, or 100% sequence identity to SEQ ID NO: 12, and/or
a light chain having at least 85%, 90%, 91%, 92%, 93%, 94%, 95%, 96%, 97%, 98%, 99%, or 100% sequence identity to SEQ ID NO: 13.

In some embodiments, provided is the anti-ROR1 antibody according to any one of the above, which comprises: a heavy chain set forth in SEQ ID NO: 12 and a light chain set forth in SEQ ID NO: 13.
In some embodiments, provided is the anti-ROR1 antibody according to any one of the above, wherein the anti-ROR1 antibody binds to human ROR1 or an epitope thereof with a KD of less than $1 \times 10^{-8}$ M, and the KD is measured by surface plasmon resonance.
In some embodiments, the present disclosure further provides an isolated anti-ROR1 antibody, wherein the antibody competes for binding to human ROR1 with the anti-ROR1 antibody according to any one of the above.
In some embodiments, the present disclosure further provides an isolated nucleic acid molecule encoding the anti-ROR1 antibody according to any one of the above.
In some embodiments, the present disclosure further provides a host cell comprising the nucleic acid molecule according to any one of the above.
In some embodiments, the present disclosure further provides a pharmaceutical composition comprising a therapeutically effective amount of the anti-ROR1 antibody according to any one of the above, or the nucleic acid molecule described above, and one or more pharmaceutically acceptable carriers, diluents, or excipients.
In some embodiments, the present disclosure further provides a method for immunodetection or determination of ROR1, the method comprising a step of contacting a subject or a sample from the subject using the anti-ROR1 antibody according to any one of the above.
In some embodiments, the present disclosure further provides an immunoconjugate or a pharmaceutically acceptable salt thereof, which comprises the anti-ROR1 antibody according to any one of the above and an effector, wherein the effector is conjugated to the anti-ROR1 antibody. Preferably, the effector is selected from the group consisting of: a radioisotope, an anti-tumor agent, an immunomodulator, a biological response modifier, a lectin, a cytotoxic drug, a chromophore, a fluorophore, a chemiluminescent compound, an enzyme, a metal ion, and any combination thereof.
In some embodiments, provided is the immunoconjugate or the pharmaceutically acceptable salt thereof described above, wherein the structure of the immunoconjugate is shown below:

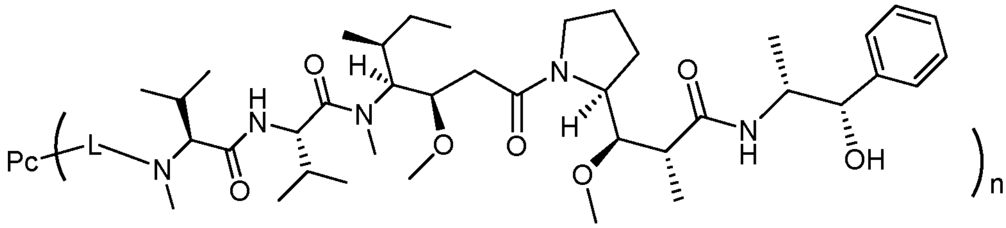

wherein:

n is an integer or a decimal from 1 to 10; preferably, n is an integer or a decimal from 1 to 8;
Pc is the anti-ROR1 antibody according to any one of the above; and
L is a linker unit.

In some embodiments, provided is the immunoconjugate or the pharmaceutically acceptable salt thereof described above, wherein the structure of the immunoconjugate is shown below:

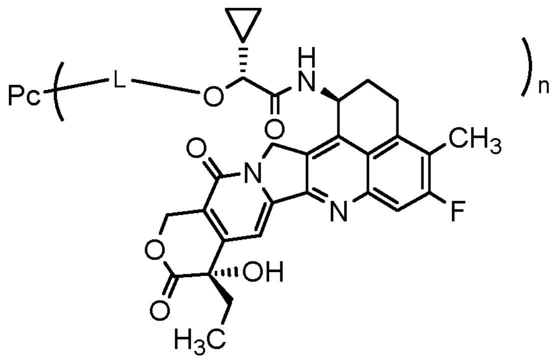

wherein:

n is an integer or a decimal from 1 to 10; preferably, n is an integer or a decimal from 1 to 8;
Pc is the anti-ROR1 antibody according to any one of the above; and
L is a linker unit.

In some embodiments, provided is the immunoconjugate or the pharmaceutically acceptable salt thereof according to any one of the above, wherein n is an integer or a decimal from 3 to 5, preferably 4.

In some embodiments, provided is the immunoconjugate or the pharmaceutically acceptable salt thereof according to any one of the above, wherein n is an integer or a decimal from 6 to 8, such as, but not limited to, 7.1, 7.2, 7.3, 7.4, 7.5, 7.6, 7.7, 7.8, 7.9, 8.0, or a range between any two of the values described above.

In some specific embodiments, provided is the immunoconjugate or the pharmaceutically acceptable salt thereof according to any one of the above, wherein n is 8.

The skilled person understands that in some specific embodiments, n is embodied in the form of a DAR value. The DAR value is a measured value, and therefore, when reference is made in the present disclosure to a particular n value (or a DAR value), it should be understood to encompass a suitable error range, the size of which can be determined by the skilled person according to the measurement system.

In some specific embodiments, provided is the immunoconjugate or the pharmaceutically acceptable salt thereof according to any one of the above, wherein n is 7.39 $\pm$ error.

In some embodiments, provided is the immunoconjugate or the pharmaceutically acceptable salt thereof according to any one of the above, wherein linker unit -L- is $-L^1-L^2-L^3-L^4-$, wherein

$L^1$ is selected from the group consisting of -(succinimidin-3-yl-*N*)-W-C(O)-, $-CH_2-C(O)-NR^1-W-C(O)-$, or -C(O)-W-C(O)-, wherein W is selected from the group consisting of $C_{1-8}$ alkyl or $C_{1-6}$ alkyl-cycloalkyl;
$L^2$ is selected from the group consisting of $-NR^2(CH_2CH_2O)p^1CH_2CH_2C(O)-$, $-NR^2(CH_2CH_2O)p^1CH_2C(O)-$, $-S(CH_2)p^1C(O)-$, or a chemical bond, wherein $p^1$ is an integer from 1 to 20; preferably, a chemical bond;
$L^3$ is a peptide residue consisting of 2 to 7 amino acid residues, wherein the amino acid residues are selected from

the group consisting of amino acid residues formed from amino acids of phenylalanine (F), glycine (G), valine (V), lysine (K), citrulline, serine (S), glutamic acid (E), and aspartic acid (D);
$L^4$ is selected from the group consisting of $-NR^3(CR^4R^5)_t-$, $-C(O)NR^3-$, $-C(O)NR^3(CH_2)_t-$, or a PAB group, wherein t is an integer from 1 to 6;
$R^1$, $R^2$, and $R^3$ are identical or different and are each independently selected from the group consisting of a hydrogen atom, alkyl, haloalkyl, deuterated alkyl, and hydroxyalkyl;
$R^4$ and $R^5$ are identical or different and are each independently selected from the group consisting of a hydrogen atom, halogen, alkyl, haloalkyl, deuterated alkyl, and hydroxyalkyl.

In some embodiments, provided is the immunoconjugate or the pharmaceutically acceptable salt thereof according to any one of the above, wherein $L^3$ is a dipeptide residue; preferably valine-citrulline (VC).

In some embodiments, provided is the immunoconjugate or the pharmaceutically acceptable salt thereof according to any one of the above, wherein $L^3$ is a tetrapeptide residue; preferably GGFG.

In some embodiments, provided is the immunoconjugate or the pharmaceutically acceptable salt thereof according to any one of the above, wherein $L^4$ is $-NR^3(CR^4R^5)t-$, wherein $R^3$, $R^4$, or $R^5$ are identical or different and are each independently a hydrogen atom or $C_{1-6}$ alkyl, and t is 1 or 2.

In some embodiments, provided is the immunoconjugate or the pharmaceutically acceptable salt thereof according to any one of the above, wherein $L^4$ is a PAB group. In some embodiments, provided is the immunoconjugate or the pharmaceutically acceptable salt thereof according to any one of the above, wherein the structure of the immunoconjugate is selected from the group consisting of:

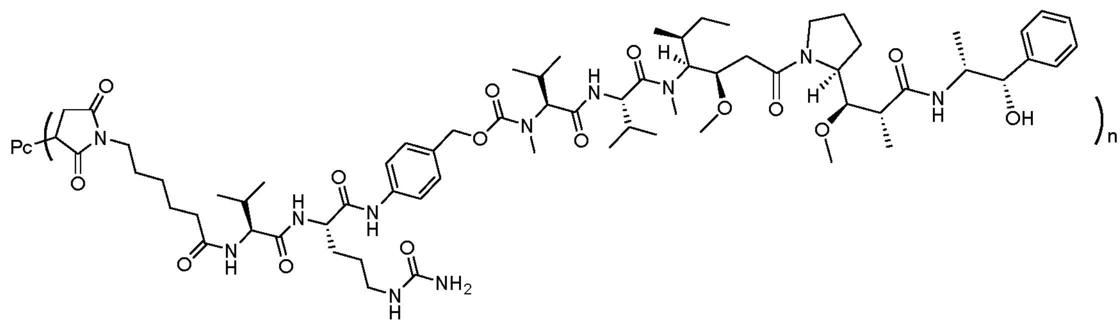

and

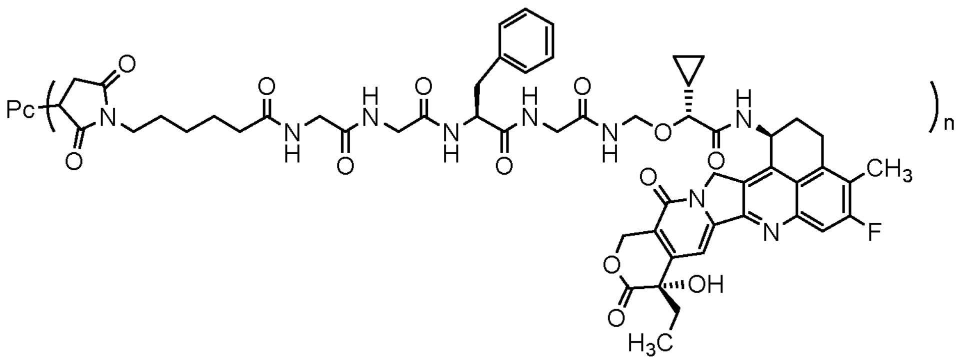

wherein:
n and Pc are as defined above.

In some embodiments, the structure of the immunoconjugate or the pharmaceutically acceptable salt thereof according to any one of the above is shown below:

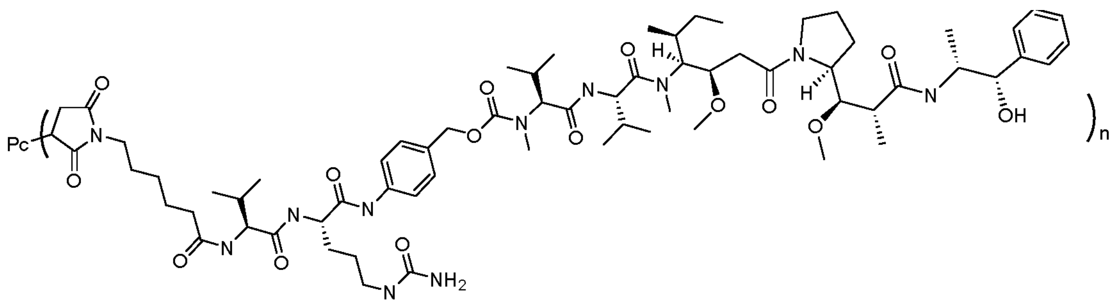

wherein:

n is an integer or a decimal from 1 to 10; preferably, n is an integer or a decimal from 1 to 8;
Pc is an anti-ROR1 antibody comprising a heavy chain set forth in SEQ ID NO: 12 and a light chain set forth in SEQ ID NO: 13.

In some embodiments, the structure of the immunoconjugate or the pharmaceutically acceptable salt thereof according to any one of the above is shown below:

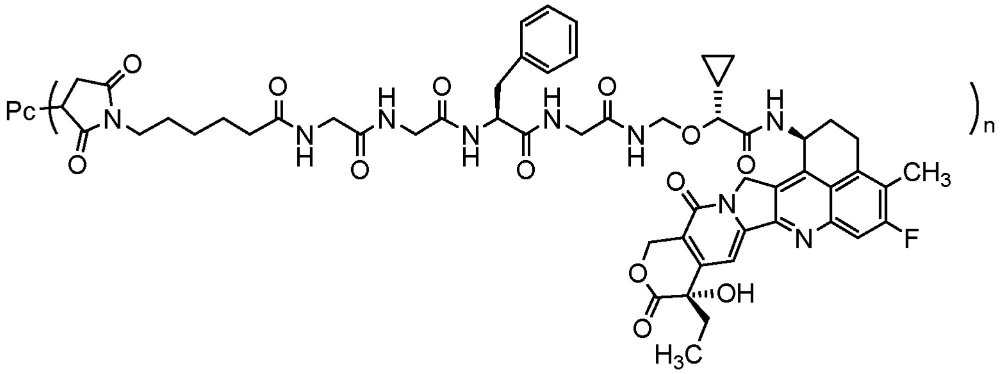

wherein:

n is an integer or a decimal from 1 to 10; preferably, n is an integer or a decimal from 1 to 8;
Pc is an anti-ROR1 antibody comprising a heavy chain set forth in SEQ ID NO: 12 and a light chain set forth in SEQ ID NO: 13.

In some embodiments, provided is the immunoconjugate or the pharmaceutically acceptable salt thereof according to any one of the above, wherein n is an integer or a decimal from 3 to 5.
In some embodiments, n is selected from an average value of 1 to 10, or 1 to 8, or 2 to 8, or 2 to 7, or 2 to 4, or 3 to 8, or 3 to 7, or 3 to 6, or 4 to 7, or 4 to 6, or 4 to 5. In some embodiments, n is an average value calculated from one or more of 1, 2, 3, 4, 5, 6, 7, 8, 9, and 10. In some embodiments, n is preferably from 6 to 8, more preferably an average value of 8.
In some embodiments, the present disclosure further provides a pharmaceutical composition comprising the anti-ROR1 antibody according to any one of the above, or the nucleic acid molecule according to any one of the above, or the immunoconjugate or the pharmaceutically acceptable salt thereof according to any one of the above, and one or more pharmaceutically acceptable excipients, diluents, or carriers.
In some embodiments, the present disclosure further provides use of the anti-ROR1 antibody according to any one of the above, or the nucleic acid molecule according to any one of the above, or the immunoconjugate or the pharmaceutically acceptable salt thereof according to any one of the above, or the pharmaceutical composition according to any one of the above, in the preparation of a drug for treating a ROR1-mediated disease or disorder.
In another aspect, the present disclosure provides use of the immunoconjugate or the pharmaceutically acceptable salt thereof according to any one of the above or the pharmaceutical composition comprising same in the preparation of a drug for treating a ROR1-mediated disease or disorder. In some embodiments, the ROR1-mediated disease or disorder is a cancer with high, medium, or low expression of ROR1.
In some embodiments, the present disclosure further provides use of the anti-ROR1 antibody according to any

one of the above, or the nucleic acid molecule according to any one of the above, or the immunoconjugate or the pharmaceutically acceptable salt thereof according to any one of the above, or the pharmaceutical composition according to any one of the above in the preparation of a drug for treating a tumor or cancer, wherein the tumor or cancer is selected from the group consisting of breast cancer, pancreatic cancer, lung cancer, esophageal cancer, non-small cell lung cancer, laryngeal tumors, sarcoma, pharyngeal tumors, oral tumors, gastric cancer, ovarian cancer, prostate cancer, bladder cancer, colorectal cancer, lymphoma and leukemia.

In some embodiments, the present disclosure further provides a kit comprising the anti-ROR1 antibody according to any one of the above, or the nucleic acid molecule according to any one of the above, or the immunoconjugate or the pharmaceutically acceptable salt thereof according to any one of the above, or the pharmaceutical composition according to any one of the above.

In another aspect, the present disclosure provides a pharmaceutical composition comprising the anti-ROR1 antibody, the immunoconjugate or the pharmaceutically acceptable salt thereof according to any one of the above, and one or more pharmaceutically acceptable excipients, diluents, or carriers. In some embodiments, a unit dose of the pharmaceutical composition comprises 0.1 mg-3000 mg or 1 mg-1000 mg of the anti-ROR1 antibody described above or the immunoconjugate described above.

In another aspect, the present disclosure provides use of the immunoconjugate or the pharmaceutically acceptable salt thereof or the pharmaceutical composition comprising same according to any one of the above as a drug.

In some embodiments, the present disclosure further provides a method for preventing or treating a disease or disorder, the method comprising administering to a subject a therapeutically effective amount of the anti-ROR1 antibody according to any one of the above, or the nucleic acid molecule according to any one of the above, or the immunoconjugate or the pharmaceutically acceptable salt thereof according to any one of the above, or the pharmaceutical composition according to any one of the above. In some embodiments, the disease or disorder is a disease or disorder associated with ROR1.

In another aspect, the present disclosure further relates to a method for treating a tumor, the method comprising administering to a subject in need thereof a therapeutically effective dose of the immunoconjugate or the pharmaceutically acceptable salt thereof or the pharmaceutical composition comprising same according to any one of the above; in some embodiments, the tumor is a cancer associated with high, medium, or low expression of ROR1.

In another aspect, the present disclosure further relates to a method for treating a tumor or cancer, the method comprising administering to a subject in need thereof a therapeutically effective dose of the immunoconjugate or the pharmaceutically acceptable salt thereof or the pharmaceutical composition comprising same according to any one of the above; in some embodiments, the tumor and cancer are selected from the group consisting of breast cancer, pancreatic cancer, lung cancer, esophageal cancer, non-small cell lung cancer, laryngeal tumors, sarcoma, pharyngeal tumors, oral tumors, gastric cancer, ovarian cancer, prostate cancer, bladder cancer, colorectal cancer, lymphoma and leukemia.

In another aspect, the present disclosure further provides the anti-ROR1 antibody or the immunoconjugate thereof according to any one of the above as a drug, in some embodiments, as a drug for treating cancer or a tumor, preferably as a drug for treating ROR1-mediated cancer.

The active compound (e.g., the anti-ROR1 antibody or the immunoconjugate thereof of the present disclosure) may be formulated into a form suitable for administration by any suitable route. The unit dose of the compound or composition of the present disclosure may be expressed in the form of a tablet, a capsule, a cachet, a vial, a powder, a granule, a lozenge, a suppository, a regenerating powder, or a liquid formulation.

The dose of the compound or composition used in the treatment method of the present disclosure generally varies depending on the severity of the disease, the weight of the subject, and the relative efficacy of the compound. However, as a general instruction, a suitable unit dose may be 0.1 mg to 1000 mg.

The pharmaceutical composition of the present disclosure may comprise, in addition to the active compound, one or more excipients selected from the group consisting of a filler, a diluent, a binder, a wetting agent, a disintegrant, an excipient and the like. Depending on the method of administration, the composition may comprise 0.1 wt.% to 99 wt.% of the active compound.

The anti-ROR1 antibody provided by the present disclosure has good affinity for a cell surface antigen and good endocytosis efficiency. The anti-ROR1 antibody immunoconjugate provided by the present disclosure has very high tumor inhibition efficiency, and has better efficacy and lower toxic and side effects in animals.

## Detailed Description of the Invention

Terminology

Unless otherwise defined, all technical and scientific terms used herein have the same meaning as commonly understood by those of ordinary skill in the art to which the present disclosure belongs. Although any methods and

materials similar or equivalent to those described herein can also be used to implement or test the present disclosure, preferred methods and materials are described herein. In describing and claiming the present disclosure, the following terms are used in accordance with the definitions below.

When a trade name is used in the present disclosure, it is intended to include the formulation of the commercial product under the trade name, and the drug and active drug component of the commercial product under the trade name.

Unless otherwise stated, the terms used in the specification and claims have the following meanings.

The three-letter and single-letter codes for amino acids used herein are as described in J. biol. chem, 243, p3558 (1968).

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a structure different from the general chemical structure of amino acids, but function in a manner similar to naturally occurring amino acids.

The term "antibody" is used herein in the broadest sense and encompasses a variety of antibody structures, including but not limited to monoclonal antibodies, polyclonal antibodies, multispecific antibodies (e.g., bispecific antibodies), and full-length antibodies, and antibody fragments (or antigen-binding fragments, or antigen-binding moieties) so long as they exhibit the desired antigen-binding activity.

"Native antibody" refers to a naturally occurring immunoglobulin molecule. For example, a native IgG antibody is a heterotetrameric glycoprotein of about 150,000 Daltons composed of two light chains and two heavy chains linked by a disulfide bond. From N-terminus to C-terminus, each heavy chain has one variable region (VH), also known as variable heavy domain or heavy chain variable domain, followed by three constant domains (CH1, CH2, and CH3). Similarly, from the N-terminus to the C-terminus, each light chain has one variable region (VL), also known as variable light domain or light chain variable domain, followed by one constant light domain (also known as a light chain constant region, CL).

The term "full-length antibody", "intact antibody", and "whole antibody" are used herein interchangeably and refer to an antibody comprising a substantially similar structure to a native antibody structure or whose heavy chains have an Fc region as defined herein.

"Isolated antibody" is an antibody that has been separated from components of its natural environment. In the present disclosure, in some embodiments, the antibody may be purified to a purity of greater than 90% or a purity of 99%. In some embodiments, the antibody is purified and assayed by methods such as electrophoresis (e.g., SDS-PAGE, isoelectric focusing (IEF), capillary electrophoresis) or chromatography (e.g., ion exchange or reversed-phase HPLC).

The term "variable region" or "variable domain" refers to a domain in antibody heavy and/or light chains that is involved in the binding of the antibody to an antigen. The VH and VL of a native IgG antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues in variable domains. A VH comprises 3 CDR regions: HCDR1, HCDR2, and HCDR3; a VL comprises 3 CDR regions: LCDR1, LCDR2, and LCDR3. Each VH and VL is composed of three CDRs and four FRs arranged from the amino terminus to the carboxyl terminus in the following order: FR1, CDR1, FR2, CDR2, FR3, CDR3, and FR4. A single VH or VL may be sufficient to provide antigen-binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol., Oct. 16, 2018; 9: 2278), and the like. The corresponding relationships among the various numbering schemes are well known to those skilled in the art, illustratively, as shown in Table 1 below.

Table 1. Relationships among CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |

(continued)

| CDR | IMGT | Kabat | AbM | Chothia | Contact |
|---|---|---|---|---|---|
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The term "light chain" includes the variable region domain VL and the constant region domain CL. VL is at the amino terminus of a light chain. Light chains include κ and λ chains.

The term "heavy chain" includes the variable region domain VH and the three constant region domains CH1, CH2 and CH3. VH is at the amino terminus of a heavy chain and the CH domains are at the carboxy terminus, with CH3 being closest to the carboxy terminus of a polypeptide. A heavy chain may be of any isotype, including IgG (including IgG1, IgG2, IgG3 and IgG4 subtypes), IgA (including IgA1 and IgA2 subtypes), IgM and IgE.

The term "antibody fragment" refers to a molecule different from an intact antibody, which comprises a moiety of an intact antibody that binds to an antigen to which the intact antibody binds. Antibody fragments include, but are not limited to, Fv, Fab, Fab', Fab'-SH, $F(ab')_2$, single-domain antibodies, single-chain Fab (scFab), diabodies, linear antibodies, single-chain antibody molecules (e.g., scFv), and multispecific antibodies formed from antibody fragments.

The term "Fc region" or "fragment crystallizable region" is used to define the C-terminal region of the heavy chain of an antibody, including native sequence Fc regions and modified Fc regions. In some embodiments, the Fc region of the human IgG heavy chain is defined as extending from the amino acid residue at position Cys226 or from Pro230 to its carboxyl terminus. The boundaries of the Fc region of the heavy chain of an antibody may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Thus, in some embodiments, a composition of intact antibodies may comprise antibody populations with all K447 residues and/or G446 + K447 residues removed. In some embodiments, a composition of intact antibodies may comprise antibody populations without K447 residue and/or G446 + K447 residue removed. In some embodiments, a composition of intact antibodies has a mixture of antibodies with and without K447 residues and/or G446 + K447 residues. Suitable native sequence Fc regions for the antibodies described herein include human IgG1, IgG2 (IgG2A, IgG2B), IgG3, and IgG4. Unless otherwise specified herein, the numbering of amino acid residues in the Fc region or constant region conforms to the EU numbering scheme, also known as the EU index, as described in Kabat et al., Sequences of Proteins of Immunological Interest, 5th ed., Public Health Service, National Institutes of Health, Bethesda, MD, 1991.

The term "human antibody" refers to an antibody in which the variable and constant regions are human sequences. The term encompasses antibodies that are derived from human genes but have, for example, sequences that have been altered to, e.g., reduce possible immunogenicity, increase affinity, eliminate cysteines or glycosylation sites that may cause undesired folding. The term encompasses antibodies recombinantly produced in non-human cells (that may confer glycosylation not characteristic of human cells). The term also encompasses antibodies that are produced in transgenic mice comprising human immunoglobulin heavy and light chain loci. The meaning of the human antibody specifically excludes humanized antibodies comprising non-human antigen-binding residues.

The term "affinity-matured" antibody refers to an antibody with one or more alterations in one or more CDRs thereof which result in an improvement in the affinity of the antibody for antigens, compared to the parent antibody. In some embodiments, an affinity-matured antibody has nanomolar or even picomolar affinity for the target antigen. Affinity-matured antibodies can be produced using methods known in the art. Marks et al., Bio/Technology 10:779-783 (1992) described affinity maturation by VH and VL domain shuffling. The following documents describe random mutagenesis of CDR and/or framework residues: Barbas et al., PNAS, 91:3809-3813 (1994); Schier et al., Gene 169:147-155 (1995); Yelton et al., J.Immunol. 155:1994-2004 (1995); Jackson et al., J.Immunol. 154 (7):3310-9 (1995), and Hawkins et al., J. Mol. Biol. 226:889-896 (1992).

The term "monoclonal antibody" refers to a population of substantially homogeneous antibodies, that is, the amino acid sequences of the antibody molecules comprised in the population are identical, except for a small number of natural mutations that may exist. In contrast, polyclonal antibody formulations generally comprise a plurality of different antibodies having different amino acid sequences in their variable domains, which are generally specific for different epitopes. "Monoclonal" refers to the characteristics of an antibody obtained from a substantially homogeneous population of antibodies, and should not be construed as requiring the production of the antibody by any particular method. For example, monoclonal antibodies can be prepared by the hybridoma method described in Kohler et al., (1975) Nature 256:495, or by recombinant DNA methods (see, e.g., U.S. Patent No. 4,816,567). Alternatively, "monoclonal antibodies" can be isolated from phage antibody libraries using the techniques described in Clackson et al., (1991) Nature 352:

624-628 and Marks et al., (1991) J. Mol. Biol. 222:581-597. Or see Presta (2005) J. Allergy Clin. Immunol. 116:731, or "monoclonal antibodies" can be prepared using methods utilizing transgenic animals containing all or part of the human immunoglobulin loci.

The term "antigen" refers to a molecule or a portion of a molecule that is capable of being selectively bound by an antibody. An antigen may have one or more epitopes capable of interacting with different antibodies.

The term "epitope" refers to an area or region on an antigen that is capable of specifically binding to an antibody. Epitopes can be formed from contiguous strings of amino acids (linear epitopes) or comprise non-contiguous amino acids (conformational epitopes), e.g., coming in spatial proximity due to the folding of the antigen (i.e., by the tertiary folding of the antigen). The difference between the conformational epitope and the linear epitope is that in the presence of denaturing solvents, the binding of the antibody to the conformational epitope is undetectable. An epitope comprises at least 3, at least 4, at least 5, at least 6, at least 7, or 8-10 amino acids in a unique spatial conformation.

Screening for antibodies that bind to particular epitopes (i.e., those that bind to identical epitopes) can be performed using methods well known in the art, including but not limited to, alanine scanning, peptide blotting (see Meth. Mol. Biol. 248 (2004) 443-463), peptide cleavage analysis, epitope excision, epitope extraction, chemical modification of the antigen (see Prot. Sci. 9 (2000) 487-496), and cross-blocking (see "Antibodies", Harlow and Lane (Cold Spring Harbor Press, Cold Spring Harb., NY)).

"An antibody that binds to the same epitope" as a reference antibody or "an antibody that competes for binding with" a reference antibody refers to an antibody that blocks binding of the reference antibody to an antigen by 50% or more, or an antibody whose binding to an antigen is blocked by 50% or more by the reference antibody, in a competition assay. For example, to determine whether the test antibody binds to the same epitope as the reference antibody, the reference antibody is allowed to bind to the antigen under saturating conditions. After removal of excess reference antibody, the ability of the test antibody to bind to the antigen is assessed. To confirm whether the test antibody binds to the same epitope or is just hampered from binding by steric reasons, conventional experimentation can be used (e.g., peptide mutation and binding analyses using ELISA, RIA, surface plasmon resonance, flow cytometry or any other quantitative or qualitative antibody-binding assay available in the art). This assay should be carried out in two set-ups, i.e. with both of the antibodies being saturating antibodies. If, in both set-ups, only the first (saturating) antibody is capable of binding to the antigen, then it can be concluded that the test antibody and the reference antibody compete for binding to the antigen.

In some embodiments, two antibodies are considered to bind to the same epitope or an overlapping epitope if a 1-, 5-, 10-, 20- or 100-fold excess of one antibody inhibits binding of the other by at least 50%, at least 75%, at least 90% or even 99% or more as measured in a competitive binding assay (see, e.g., Junghans et al., Cancer Res. 50 (1990) 1495-1502).

In some embodiments, two antibodies are considered to bind to the "same epitope" if essentially all amino acid mutations in the antigen that reduce or eliminate binding of one antibody reduce or eliminate binding of the other. Two antibodies are considered to have "overlapping epitopes" if only some of the mutations reduce or eliminate binding of the other.

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to an internal binding affinity that reflects the 1: 1 interaction between members of a binding pair (e.g., an antibody and an antigen). The affinity of a molecule X for its ligand Y can be generally expressed by the equilibrium dissociation constant (KD). Affinity can be determined by conventional methods known in the art, including those described herein. As used herein, the term "kassoc" or "ka" refers to the association rate of a particular antibody-antigen interaction, while the term "kdis" or "kd" refers to the dissociation rate of a particular antibody-antigen interaction. The term "KD" refers to the equilibrium dissociation constant, which is obtained from the ratio of kd to ka (i.e., kd/ka) and denoted by a molar concentration (M). The KD value of an antibody can be determined using methods well known in the art. For example, surface plasmon resonance is determined using a biosensing system such as a system, or affinity in a solution is determined by solution equilibrium titration (SET).

The term "specifically bind", "specific binding" or "binds" refers to antibody binding to an antigen or an epitope thereof with greater affinity than to other antigens or epitopes. Typically, the antibody binds to the antigen or the epitope thereof with an equilibrium dissociation constant (KD) of about $1 \times 10^{-7}$ M or less (e.g., about $1 \times 10^{-8}$ M or less, about $1 \times 10^{-9}$ M or less). In some embodiments, the KD for the binding of an antibody to an antigen is 10% or less (e.g., 1%) of the KD for the binding of the antibody to a non-specific antigen (e.g., BSA or casein). KD may be determined using known methods in the art, for example, by a BIACORE® surface plasmon resonance assay. However, an antibody that specifically binds to an antigen or an epitope thereof may have cross-reactivity to other related antigens, e.g., to corresponding antigens from other species (homologous), such as humans or monkeys, e.g., *Macaca fascicularis* (cynomolgus, cyno), *Pan troglodytes* (chimpanzee, chimp), or *Callithrix jacchus* (commonmarmoset, marmoset).

The terms "anti-ROR1 antibody" and "antibody that binds to ROR1" refer to an antibody that is capable of binding to ROR1 with sufficient affinity. In certain embodiments, the antibody that binds to ROR1 has an equilibrium

dissociation constant (KD) of < about 1 μM, < about 100 nM, < about 10 nM, < about 1 nM, < about 0.1 nM, < about 0.01 nM, or < about 0.001 nM (e.g., $10^{-8}$ M or less, e.g., $10^{-8}$ M to $10^{-9}$ M, e.g., $10^{-9}$ M or less). In certain embodiments, the anti-ROR1 antibody binds to a conserved ROR1 epitope of ROR1 from different species.

The term "antibody-dependent cell-mediated cytotoxicity" or "ADCC" refers to a form of cytotoxicity in which secreted Ig bound onto Fc receptors (FcRs) present on certain cytotoxic cells (e.g., natural killer (NK) cells, neutrophils and macrophages) enables these cytotoxic effector cells to specifically bind to antigen-bearing target cells and subsequently kill the target cells with cytotoxins. The antibodies "arm" the cytotoxic cells and are essential for such killing. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol, 9:457-92 (1991). To assess the ADCC activity of a target molecule, an *in vitro* ADCC assay, such as that described in U.S. Patent No. 5500362 or 5821337 may be performed. Useful effector cells for such assays include peripheral blood mononuclear cells (PBMCs) and natural killer (NK) cells. Alternatively, the ADCC activity of the target molecule may be assessed *in vivo,* e.g., in an animal model (such as that disclosed in Clynes et al., (USA) 95:652-656 (1998)). The term "antibody-dependent cellular phagocytosis" ("ADCP") refers to a mechanism by which antibody-coated target cells are eliminated by internalization of phagocytic cells such as macrophages or dendritic cells.

The term "complement-dependent cytotoxicity" or "CDC" refers to a mechanism for inducing cell death in which the Fc effector domain of a target-binding antibody binds to and activates the complement component C1q, and C1q then activates the complement cascade, resulting in the death of the target cell. Activation of a complement may also result in the deposition of complement components on the surface of target cells that promote CDC by binding to complement receptors on leukocytes (e.g., CR3).

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. "Nucleic acid encoding the anti-ROR1 antibody" refers to one or more nucleic acid molecules encoding the antibody heavy and light chains (or fragments thereof).

Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes8:91-98, 1994).

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers.

The term "naked antibody" refers to an antibody that is not conjugated to a heterologous module (e.g., a cytotoxic module) or a radioactive label. A naked antibody may be present in a pharmaceutical formulation.

The term "identity", "sequence identity" or "amino acid sequence identity" refers to the degree (percentage) to which the amino acids/nucleic acids of two sequences are identical at equivalent positions, when the two sequences are optimally aligned, with gaps introduced as necessary to achieve the maximum percent sequence identity, and without considering any conservative substitutions as part of the sequence identity. To determine percent sequence identity, alignment can be achieved using methods that are well known in the art, such as BLAST, BLAST-2, ALIGN, ALIGN-2 or Megalign (DNASTAR). Those skilled in the art can determine parameters suitable for measuring alignment, including any algorithms required to achieve maximum alignment of the full length of the aligned sequences.

The term "conservatively modified variant" or "conservative substitution" refers to substitutions of amino acids in a protein with other amino acids having similar characteristics (e.g. charge, side-chain size, hydrophobicity/hydrophilicity, backbone conformation and rigidity), such that the changes can be often made without altering the biological activity of the protein. Those skilled in the art recognize that, in general, single amino acid substitutions in non-essential regions of a polypeptide do not substantially alter biological activity (see, e.g., Watson et al., (1987) Molecular Biology of the Gene, The Benjamin/Cummings Pub. Co., p. 224 (4th Ed.)). The term "conservatively modified variant", when applied to nucleic acid sequences, refers to those nucleic acids which encode identical or essentially identical amino acid sequences, or where the nucleic acid does not encode an amino acid sequence, to essentially identical sequences.

Because of the degeneracy of the genetic code, a number of functionally identical nucleic acids encode any given protein. For example, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations", which are one species of conservatively modified variations. Every nucleic acid sequence herein which encodes a polypeptide also describes every possible silent variation of the nucleic acid. One of skill will recognize that each codon in a nucleic acid (except AUG, which is normally the only codon for methionine, and TGG, which is normally the only codon for tryptophan) can be modified to produce a functionally identical molecule. Thus, each silent variation of a nucleic acid that encodes a polypeptide is implicit in each sequence described.

The term "expression vector" or "expression construct" refers to a vector that is suitable for transforming a host cell and comprises a nucleic acid sequence that directs and/or controls the expression of one or more heterologous coding regions operably linked thereto. Expression constructs may include, but are not limited to, sequences that affect or control transcription and translation and affect RNA splicing of a coding region operably linked thereto in the presence of an intron.

As used herein, "operably linked" means that the components to which the term is applied are in a relationship that allows them to carry out their inherent functions under suitable conditions. For example, a control sequence in a vector that is "operably linked" to a protein-coding sequence is ligated thereto so that expression of the protein-coding sequence is achieved under conditions compatible with the transcriptional activity of the control sequences.

The terms "host cell", "host cell line", and "host cell culture" are used interchangeably and refer to cells into which exogenous nucleic acids have been introduced, including progeny of such cells. Host cells include "transformants" and "transformed cells", which include primary transformed cells and progeny derived therefrom, regardless of the number of passages. Progeny may not be exactly the same as parent cells in terms of nucleic acid content and may contain mutations. The term includes mutant progeny that have the identical function or biological activity as the cells screened or selected from the initially transformed cells. Host cells include prokaryotic and eukaryotic host cells, wherein eukaryotic host cells include, but are not limited to, mammalian cells, insect cell lines, plant cells, and fungal cells. Exemplary host cells are as follows: Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells and HEK-293 cells, *Pichiapastoris, Pichia finlandica, Candida albicans, Aspergillus niger, Aspergillus oryzae* and *Trichoderma reesei.*

As used in the present application, the expressions "cell", "cell line" and "cell culture" are used interchangeably and include the progeny of such a cell. Thus, the words "transformant" and "transformed cell" include the primary subject cell and cultures derived therefrom, regardless of the passage number. It is also understood that not all progeny have precisely identical DNA contents due to deliberate or inadvertent mutations. Variant progeny that have the identical function or biological activity as the original transformed cell from which they were selected are included.

Methods for producing and purifying antibodies and antigen-binding fragments are well known in the art, for example, those described in chapters 5-8 and 15 of "Antibodies: A Laboratory Manual", Cold Spring Harbor Press. The antibody or the antigen-binding fragment described herein is genetically engineered to contain one or more additional human FRs in the non-human CDRs. Human FR species sequences can be obtained at the website of ImMunoGeneTics (IMGT) or from the immunoglobulin journal, 2001ISBN012441351, by alignment with the IMGT human antibody variable region germline gene database with the MOE software.

The engineered antibody or antigen-binding fragment of the present disclosure can be prepared and purified by conventional methods. For example, cDNA sequences encoding the heavy and light chains can be cloned and recombined into an expression vector. Recombinant immunoglobulin expression vectors can be stably transfected into host cells. As a more recommended prior art, mammalian expression systems will result in glycosylation of the antibody, particularly at the N-terminal site of the Fc region. Stable clones are obtained by expression of the antibody that specifically binds to human ROR1. Positive clones are expanded in a medium in a bioreactor to produce antibodies. The culture medium with the secreted antibody can be purified by conventional techniques. For example, using an A or G Sepharose FF column. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

"Isolated" refers to the separation from its naturally occurring state, and in this case means that the designated molecule is substantially free of other biomolecules, such as nucleic acids, proteins, lipids, carbohydrates, or other materials (such as cell debris and growth medium). Generally, the term "isolated" does not mean the complete absence of such materials or the absence of water, buffers or salts, unless they are present in amounts that will significantly interfere with the experimental or therapeutic use of the compounds described herein.

The term "drug" refers to a chemical substance that can alter an organism's physiology and pathological state and can be used for the prevention and treatment of diseases. The drug includes a cytotoxic drug. There is no clear

boundary between a drug and a toxic substance. The toxic substance refers to a chemical substance that has a toxic effect on organisms and can cause damage to human health even in small doses. Any drug in large doses may induce toxic responses.

The cytotoxic drug refers to a substance that inhibits or prevents cell functions and/or causes cell death or cell destruction. The cytotoxic drug can kill tumor cells in principle at a sufficiently high concentration; however, due to lack of specificity, the cytotoxic drug can cause apoptosis of normal cells while killing tumor cells, resulting in serious side effects. The cytotoxic drug includes toxins, such as small molecule toxins or enzymatically active toxins of bacterial, fungal, plant or animal origin, radioisotopes (e.g., $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic drugs, antibiotics and nucleolytic enzymes.

"Antibody-drug conjugate (ADC)" or immunoconjugate means that an antibody is linked to a drug with biological activity or cell killing activity by a linker unit.

The antibody or the antibody fragment described herein may be conjugated to an effector by any means. For example, the antibody or the antibody fragment may be chemically or recombinantly attached to the cytotoxic drug. Chemical means for preparing fusions or conjugates are known in the art and can be used to prepare immunoconjugates. The method for conjugating the antibody or the antibody fragment and the drug must be capable of linking the antibody to the drug without interfering with the ability of the antibody or the antibody fragment to bind to the target molecule. In one embodiment, both the antibody and cytotoxic drug are proteins and can be conjugated using techniques well known in the art. There are hundreds of cross-linking agents disclosed in the art that can conjugate two proteins. The cross-linking agent is generally selected based on reactive functional groups available or inserted on the antibody or cytotoxic drug. Alternatively, if no reactive groups are present, a photo-activatable cross-linking agent may be used. In some cases, it may be desirable to include a spacer between the antibody and the cytotoxic drug. Cross-linking agents known in the art include homobifunctional agents: glutaraldehyde, dimethyl adipimidate and bis(diazobenzidine), and heterobifunctional agents: m-maleimidobenzoyl-N-hydroxysuccinimide and sulfo-m-maleimidobenzoyl-N-hydroxysuccinimide. Cross-linking agents that can be used to conjugate an effector to an antibody fragment include, for example, TPCH (S-(2-thiopyridyl)-L-cysteine hydrazide) and TPMPH (S-(2-thiopyridyl)sulfhydryl-propionhydrazide). TPCH and TPMPH react on the carbohydrate moiety of the glycoprotein that had previously been oxidized by mild periodate treatment, thereby forming a hydrazone bond between the hydrazide moiety of the crosslinking agent and the aldehyde generated by periodate. The heterobifunctional cross-linking agents GMBS (N-(y-maleimidobutyryloxy)-succinimide) and SMCC (succinimidyl 4-(N-maleimido-methyl)cyclohexane) are reacted with a primary amine, thereby introducing a maleimido group onto the component. This maleimido group may then react with a sulfhydryl group on another component which may be introduced by a cross-linking agent, thereby forming a stable thioether bond between the components. If steric hindrance between the components interferes with the activity of either component, a cross-linking agent may be used to introduce a long spacer between the components, such as N-succinimidyl 3-(2-pyridyldithio)propionate (SPDP). Thus, there are many suitable cross-linking agents that may be used and selected individually depending on their effect on the yield of the optimal immunoconjugate.

Drug loading, also referred to as drug-to-antibody ratio (DAR), refers to the average number of drugs coupled to each antibody in an ADC. It may range, for example, from about 1 to about 10 drugs conjugated to each antibody, and in certain embodiments, from about 1 to about 8 drugs conjugated to each antibody, preferably selected from the group consisting of 2-8, 2-7, 2-6, 2-5, 2-4, 3-4, 3-5, 5-6, 5-7, 5-8, and 6-8 drugs conjugated to each antibody. Illustratively, the drug loading may be an average value calculated from one or more of 1, 2, 3, 4, 5, 6, 7, 8, 9, and 10. The ADC general formulas of the present disclosure include a group of immunoconjugates within a certain range as described above. In the embodiments of the present disclosure, drug loading may be represented by n and is a decimal or an integer. Drug loading can be determined by conventional methods such as UV/visible spectroscopy, mass spectrometry, ELISA assay and HPLC.

The term "linker unit" or "linker fragment" refers to a chemical structure fragment or bond, which is linked to an antibody or antigen-binding fragment thereof at one end and to a drug at the other end, and also may be linked to a drug after being linked to another linker.

The term linker may comprise one or more linker components. Exemplary linker components include 6-maleimidocaproyl ("MC"), maleimidopropionyl ("MP"), valine-citrulline ("val-cit" or "vc"), alanine-phenylalanine ("ala-phe"), *p*-aminobenzyloxycarbonyl ("PAB"), N-succinimidyl 4-(2-pyridylthio)pentanoate ("SPP"), N-succinimidyl 4-(N-maleimidomethyl)cyclohexane-1 carboxylate ("SMCC", also referred to herein as "MCC"), and N-succinimidyl(4-iodo-acetyl)aminobenzoate ("SIAB"). The linker may include stretchers, spacers and amino acid units, and may be synthesized using methods known in the art, such as those described in US2005-0238649A1. A linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers, or disulfide-containing linkers can be used (Chari et al., Cancer Research, 52: 127-131(1992); U.S. Patent No. 5208020).

Linker components include, but are not limited to:

MC = 6-maleimidocaproyl, whose structure is shown below:

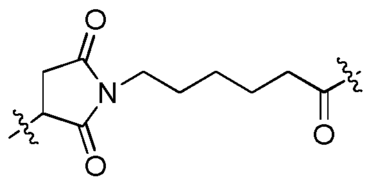

,

Val-Cit or "vc" = valine-citrulline (an exemplary dipeptide in a protease cleavable linker),
citrulline = 2-amino-5-ureidopentanoic acid,
PAB group = *p*-aminobenzyloxycarbonyl (an example of "self-immolative" linker components), whose structure is shown below:

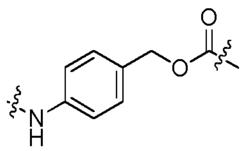

;

Me-Val-Cit = N-methyl-valine-citrulline (where the linker peptide bond has been modified to prevent it from being cleaved by cathepsin B),
MC(PEG)6-OH = maleimidocaproyl-polyethylene glycol (attachable to antibody cysteine),
SPP = N-succinimidyl 4-(2-pyridylthio)valerate,
SPDP = N-succinimidyl 3-(2-pyridyldithio)propionate,
SMCC = succinimidyl-4-(N-maleimidomethyl)cyclohexane-1-carboxylate, and
IT = iminothiolane.

In one embodiment of the present disclosure, the cytotoxic drug is conjugated to an antibody by a linker unit.
The drug loading of a conjugate may be controlled by the following non-limiting methods, including:

(1) controlling the molar ratio of a linking moiety of the drug to the monoclonal antibody,
(2) controlling reaction time and temperature, and
(3) selecting different reaction reagents.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkyl). The alkyl is preferably an alkyl group having 1 to 12 carbon atoms (i.e., $C_{1-12}$ alkyl), and more preferably an alkyl group having 1 to 6 carbon atoms (i.e., $C_{1-6}$ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, and the like. Alkyl may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.
The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). The cycloalkyl is preferably a cycloalkyl group having 3 to 12 ring atoms (i.e., 3- to 12-membered cycloalkyl), more preferably a cycloalkyl group having 3 to 8 ring atoms (i.e., 3- to 8-membered cycloalkyl), and most preferably a cycloalkyl group having 3 to 6 ring atoms (i.e., 3- to 6-membered cycloalkyl) or a cycloalkyl group having 5 ring atoms (i.e., 5-membered cycloalkyl).

Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The term "haloalkyl" refers to alkyl in which the hydrogen is substituted with one or more halogens, wherein the alkyl is as defined above.

The term "deuterated alkyl" refers to alkyl in which the hydrogen is substituted with one or more deuterium atoms, wherein the alkyl is as defined above.

The term "haloalkoxy" refers to alkoxy in which the hydrogen is substituted with one or more halogens, wherein the alkoxy is as defined above.

The term "hydroxyalkyl" refers to an alkyl group substituted with one or more hydroxy groups, wherein the alkyl is as defined above.

The term "halogen" refers to fluorine, chlorine, bromine, or iodine.

The term "hydroxy" refers to -OH.

The term "amino" refers to $-NH_2$.

The term "cyano" refers to -CN.

"Optionally" or "optional" means that the event or circumstance subsequently described may, but does not necessarily, occur, and this description includes instances where the event or circumstance occurs or does not occur. For example, "$C_{1-6}$ alkyl that is optionally substituted with a halogen or cyano" means that the halogen or cyano may, but does not necessarily, exist, and this description includes the instance where the alkyl is substituted with a halogen or cyano and the instance where the alkyl is not substituted with a halogen or cyano.

"Substituted" means that one or more, preferably 1 to 6, and more preferably 1 to 3, hydrogen atoms in the group are independently substituted with a corresponding number of substituents. Those skilled in the art can determine (experimentally or theoretically) possible or impossible substitutions without undue effort. For example, it may be unstable when an amino or hydroxy having free hydrogen is bound to a carbon atom having an unsaturated (e.g., olefinic) bond.

"Pharmaceutically acceptable salt" refers to a salt of the compound of the present disclosure, which may be selected from the group consisting of inorganic and organic salts. Such salts are safe and effective when used in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

In the present disclosure, the concentration of the immunoconjugate is expressed in protein concentration, i.e., the concentration of the antibody moiety in the immunoconjugate.

For drugs or pharmacologically active agents, the term "therapeutically effective amount" refers to an amount of a drug or an agent that is sufficient to provide the desired effect but is non-toxic. The determination of the effective amount varies from person to person. It depends on the age and general condition of a subject, as well as the particular active substance used. The appropriate effective amount in a case may be determined by those skilled in the art in light of routine tests.

The compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol and imine-enamine, lactam-lactim isomerization. An example of the lactam-lactim equilibrium is present between A and B as shown below.

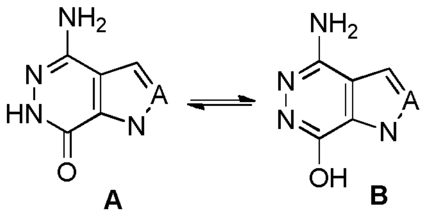

A B.

All compounds in the present disclosure can be drawn as form A or form B. All tautomeric forms are within the scope of the present disclosure. The names of the compounds do not exclude any tautomers.

"Prodrug" refers to a substance that can be converted *in vivo* under physiological conditions, e.g., by hydrolysis in blood, to generate the active prodrug compound.

The term "pharmaceutically acceptable" as used herein means that those compounds, materials, compositions,

and/or dosage forms that are, within the scope of reasonable medical judgment, suitable for use in contact with the tissues of subjects without excessive toxicity, irritation, allergic reaction, or other problems or complications, and are commensurate with a reasonable benefit/risk ratio and effective for the intended use. For the preparation of conventional pharmaceutical compositions, refer to Chinese Pharmacopoeia.

As used herein, the singular forms "a", "an" and "the" include plural references and vice versa, unless otherwise clearly defined in the context.

"About" means that it is within an acceptable error range for a particular value as determined by one of ordinary skill in the art, which will depend in part on how the value is measured or determined, i.e., the limitations of the measurement system. In the context of a particular assay, result or embodiment, "about" means that it is within one standard deviation according to the practice in the art, unless otherwise explicitly stated in the example or elsewhere in the specification.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "package insert" is used to refer to instructions generally included in commercial packages of therapeutic products, which contain information about the indications, usage, dose, administration, combination therapy, contraindications and/or warnings concerning the use of such therapeutic products.

The term "subject" or "individual" includes humans and non-human animals. Non-human animals include all vertebrates (e.g., mammals and non-mammals), non-human primates (e.g., cynomolgus monkeys), sheep, dogs, cows, chickens, amphibians, and reptiles. Unless indicated, the terms "patient" and "subject" are used interchangeably herein. As used herein, the term "cynomolgus monkey (cyno)" or "cynomolgus" refers to Macaca fascicularis. In certain embodiments, the individual or subject is a human.

The term "excipient" is an addition, besides the active ingredient, to a pharmaceutical formulation. It may also be referred to as an auxiliary material. For example, binders, fillers, disintegrants and lubricants in tablets; the matrix part in semisolid ointment and cream preparations; preservatives, antioxidants, corrigents, fragrances, cosolvents, emulsifiers, solubilizers, osmotic pressure regulators, colorants and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug’s compression moldability and the like. When the drug in the tablet form contains oily components, an absorbent is necessarily added to absorb the oily components so as to maintain a "dry" state and thus facilitate the preparation of the tablet. Examples include starch, lactose, inorganic salts of calcium, microcrystalline cellulose and the like.

The term "pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote administration to an organism and facilitate the absorption of the active ingredient so that it can exert its biological activity. The pharmaceutical composition may be in the form of a sterile injectable aqueous solution. Available and acceptable vehicles or solvents include water, Ringer’s solution and isotonic sodium chloride solution. The sterile injectable formulation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be locally injected into the bloodstream of a subject in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition may be in the form of a sterile injectable aqueous or oily suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent, e.g., a solution prepared in 1,3-butanediol. In addition, a sterile fixed oil may be conventionally used as a solvent or a suspending medium. For this purpose, any blend fixed oil including synthetic monoglycerides or diglycerides can be used. In addition, fatty acids such as oleic acid may also be used in the preparation of injections. "Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection of fluids, cells, or tissue isolated from a subject, as well as fluids, cells, or tissue present in a subject. Exemplary samples are biological fluids (such as blood; serum; serosal fluids; plasma; lymph; urine; saliva; cystic fluids; tears; excretions; sputum; mucosal secretions of secretory tissue and organs; vaginal

secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned media); lavage fluids; and the like), tissue biopsy samples, fine needle punctures, surgically excised tissues, organ cultures, or cell cultures.

The term "pharmaceutically acceptable salt" refers to a salt of the immunoconjugate of the present disclosure, or a salt of the compound described in the present disclosure. Such salts are safe and effective when used in the body of a mammal and possess the required biological activity. The antibody-drug conjugates of the present disclosure contain at least one amino group and therefore can form salts with acids.

"Treatment" or "treat" (and grammatical variations thereof) refer to clinical intervention in an attempt to alter the natural course of the treated individual, which may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antibody of the present disclosure is used to delay the development of or slow the progression of a disease.

"Effective amount" is generally an amount sufficient to reduce the severity and/or frequency of symptoms, eliminate symptoms and/or underlying causes, prevent the appearance of symptoms and/or their underlying causes, and/or ameliorate or alleviate damage (e.g., lung disease) caused by or associated with a disease state. In some embodiments, the effective amount is a therapeutically effective amount or a prophylactically effective amount. "Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. Complete treatment or prevention does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on the following factors: e.g., the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a subject.

One or more embodiments of the present disclosure are described in detail in the specification above. Although any methods and materials similar or identical to those described herein can be used in the practice or testing of the present disclosure, the preferred methods and materials are described below. Other features, objects and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

## DETAILED DESCRIPTION

The present invention is further described below with reference to examples, but these examples are not intended to limit the scope of the present invention.

Experimental methods without specific conditions indicated in the examples or test examples of the present disclosure were generally conducted under conventional conditions or conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific origins indicated are commercially available conventional reagents.

## I. Antibody Examples

### Example 1: Construction of Cell Strain Highly Expressing ROR1

PBABE-ROR1 lentiviral expression vector plasmids, pVSV-G and pGag-pol lentiviral system packaging vectors were transfected into viral packaging cells 293T (Cell Bank of Chinese Academy of Sciences, SCSP-502) using Lipofectamine 3000 (ThermoFisher, L3000001) transfection reagent. The medium supernatant containing viruses was collected, filtered, and centrifuged at ultra-high speed. Chinese hamster ovary cells CHO-K1 (Cell Bank of Chinese Academy of Sciences, SCSP-507) were allowed to be infected with the concentrated virus, screened using puromycin for two to three weeks, and subjected to FACS single-cell sorting.

According to the ROR1 expression level on the surface of CHO-K1 cells infected with lentivirus determined by FACS, a monoclonal cell strain with a high ROR1 expression level was selected and designated ROR1-CHO-K1. The selected monoclonal cell strains were expanded and preserved by freezing for subsequent experiments.

Amino acid sequence of human ROR1 (UniProtKB - Q01973)

MHRPRRRGTRPPLLALLAALLLAARGAAAQETELSVSAELVPTSSWNISSELNK
DSYLTLDEPMNNITTSLGQTAELHCKVSGNPPPTIRWFKNDAPVVQEPRRLSFRS
TIYGSRLRIRNLDTTDTGYFQCVATNGKEVVSSTGVLFVKFGPPPTASPGYSDEY
EEDGFCQPYRGIACARFIGNRTVYMESLHMQGEIENQITAAFTMIGTSSHLSDKC
SQFAIPSLCHYAFPYCDETSSVPKPRDLCRDECEILENVLCQTEYIFARSNPMILM
RLKLPNCEDLPQPESPEAANCIRIGIPMADPINKNHKCYNSTGVDYRGTVSVTKS
GRQCQPWNSQYPHTHTFTALRFPELNGGHSYCRNPGNQKEAPWCFTLDENFKS
DLCDIPACDSKDSKEKNKMEILYILVPSVAIPLAIALLFFFICVCRNNQKSSSAPVQ
RQPKHVRGQNVEMSMLNAYKPKSKAKELPLSAVRFMEELGECAFGKIYKGHLY
LPGMDHAQLVAIKTLKDYNNPQQWTEFQQEASLMAELHHPNIVCLLGAVTQEQ
PVCMLFEYINQGDLHEFLIMRSPHSDVGCSSDEDGTVKSSLDHGDFLHIAIQIAA
GMEYLSSHFFVHKDLAARNILIGEQLHVKISDLGLSREIYSADYYRVQSKSLLPIR
WMPPEAIMYGKFSSDSDIWSFGVVLWEIFSFGLQPYYGFSNQEVIEMVRKRQLL
PCSEDCPPRMYSLMTECWNEIPSRRPRFKDIHVRLRSWEGLSSHTSSTTPSGGNA
TTQTTSLSASPVSNLSNPRYPNYMFPSQGITPQGQIAGFIGPPIPQNQRFIPINGYPI
PPGYAAFPAAHYQPTGPPRVIQHCPPPKSRSPSSASGSTSTGHVTSLPSSGSNQEA
NIPLLPHMSIPNHPGGMGITVFGNKSQKPYKIDSKQASLLGDANIHGHTESMISA
EL

SEQ ID NO: 1.

**Example 2**: **Screening for Anti-Human ROR1 Monoclonal Antibody**

A positive clone was obtained by three rounds of panning using a fully human semi-synthetic phage antibody library and antigen-biotinylated human ROR1 (KACTUS, Cat. # ROR-HM401B) followed by phage detection by ELISA. The positive clone was sequenced. After the sequence was obtained, the positive clone was inserted into the protein expression vector Phr-IgG and expressed on HEK293 and Expi-CHO-S. After purification, FACS and endocytic activity validation assays were performed, and a ROR1 fully human antibody molecule 347 was obtained.

Heavy chain variable region of fully human ROR1 antibody molecule 347:

QVQLVQSGAEVKKPGSSVKVSCKASGYTFTDYYISWVRQGPGQGLEWMGGINAGNGNTNYAQKFQGRVTITADESTSTAYMELSSLRSEDTAVYYCASPGWDVFDIWGQGTMVTVSS

SEQ ID NO: 2

Light chain variable region of fully human ROR1 antibody molecule 347:

DIQMTQSPSPLSASVGDRVTITCQASQDISNYLNWYQQKPGKAPKLLIYDASNLETGVPSRFSGSGSGTDFTFTISSLQPEDIATYYCQQHEDLPITFGQGTRLEIK

SEQ ID NO: 3.

The CDR regions of the anti-ROR1 antibody 347 of the present disclosure determined according to the Kabat numbering scheme are as follows:

Table 2-1. CDR regions of anti-ROR1 antibody 347 according to Kabat numbering scheme

| **Antibody** 347 | Sequence | No. |
|---|---|---|
| **HCDR1** | DYYIS | SEQ ID NO: 4 |
| **HCDR2** | GINAGNGNTNYAQKFQG | SEQ ID NO: 5 |
| **HCDR3** | PGWDVFDI | SEQ ID NO: 6 |
| **LCDR1** | QASQDISNYLN | SEQ ID NO: 7 |
| **LCDR2** | DASNLET | SEQ ID NO: 8 |
| **LCDR3** | QQHEDLPIT | SEQ ID NO: 9 |

The CDR regions of the anti-ROR1 antibody 347 of the present disclosure determined according to the Chothia and IMGT numbering schemes are as follows:

Table 2-2. CDR regions of anti-ROR1 antibody 347 according to Chothia and IMGT numbering schemes

| **Antibody** 347 | Chothia sequence | Chothia No. | IMGT sequence | IMGT No. |
|---|---|---|---|---|
| **HCDR1** | GYTFTDY | SEQ ID NO: 16 | GYTFTDYY | SEQ ID NO: 22 |
| **HCDR2** | NAGNGN | SEQ ID NO: 17 | INAGNGNT | SEQ ID NO: 23 |
| **HCDR3** | PGWDVFDI | SEQ ID NO: 18 | ASPGWDVFDI | SEQ ID NO: 24 |
| **LCDR1** | QASQDISNYLN | SEQ ID NO: 19 | QDISNY | SEQ ID NO: 25 |
| **LCDR2** | DASNLET | SEQ ID NO: 20 | DAS | SEQ ID NO: 26 |
| **LCDR3** | QQHEDLPIT | SEQ ID NO: 21 | QQHEDLPIT | SEQ ID NO: 27 |

The anti-ROR1 antibody described above may further comprise an antibody heavy chain constant region and a light chain constant region; the heavy chain constant region may be selected from the group consisting of human IgG1, IgG2, IgG3, and IgG4 constant regions, and the light chain constant region may be selected from the group consisting of human antibody κ and λ chain constant regions. In the present disclosure, the antibody comprises a heavy chain constant region having the sequence set forth in SEQ ID NO: 10, and a light chain constant region having the sequence set forth in SEQ ID NO: 11:

Human IgG1 heavy chain constant region:

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK

SEQ ID NO: 10

Human κ light chain constant region:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 11.

Sequences of anti-ROR1 antibody 347 of the present disclosure:

Heavy chain of fully human ROR1 antibody 347:

QVQLVQSGAEVKKPGSSVKVSCKASGYTFTDYYISWVRQGPGQGLEWMGGIN
AGNGNTNYAQKFQGRVTITADESTSTAYMELSSLRSEDTAVYYCASPGWDVFDI
WGQGTMVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK

SEQ ID NO: 12

Light chain of fully human ROR1 antibody 347:

DIQMTQSPSPLSASVGDRVTITCQASQDISNYLNWYQQKPGKAPKLLIYDASNL
ETGVPSRFSGSGSGTDFTFTISSLQPEDIATYYCQQHEDLPITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 13.

The anti-ROR1 antibody Ab1 in WO2018237335 was used as a positive control in the present disclosure, the sequences of which are shown below:

Heavy chain of Ab1:

QVQLQESGPGLVKPSQTLSLTCTVSGYAFTAYNIHWVRQAPGQGLEWMGSFDP
YDGGSSYNQKFKDRLTISKDTSKNQVVLTMTNMDPVDTATYYCARGWYYFDY
WGHGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
ALHNHYTQKSLSLSPGK

SEQ ID NO: 14

Light chain of Ab1:

DIVMTQTPLSLPVTPGEPASISCRASKSISKYLAWYQQKPGQAPRLLIYSGSTLQS
GIPPRFSGSGYGTDFTLTINNIESEDAAYYFCQQHDESPYTFGEGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

SEQ ID NO: 15.

**II. ADC Examples**

Determination of the DAR values of ADCs

The DAR values of the ADCs of the present disclosure were calculated by RP-HPLC (reversed-phase high performance liquid chromatography), specifically as follows:

1. Determination method:

4 μL of DDT (sigma) was added to a naked antibody and an ADC test sample (at a concentration of 1 mg/mL) for reduction, and the mixture was in a water bath at 37 °C for 1 h and then transferred to an insert. Analysis was

performed on a high performance liquid chromatograph Agilent 1200, with Agilent PLRP-S 1000A 8 μm 4.6 × 250 mm selected as the chromatographic column, the column temperature at 80 °C, the DAD detector at wavelength 280 nm, the flow rate at 1 mL/min, and the injection volume at 40 μL. Comparisons were made to the spectra of the sample and the naked antibody to identify the locations of the light chain and heavy chain, and then integration was performed on the spectrum of the test sample to calculate the DAR value.

2. Preparation of solutions

1) 0.25 M DTT solution:

Example of preparation: 5.78 mg of DTT was weighed into 150 μL of purified water and completely dissolved to give 0.25 M DTT solution, which was then stored at -20 °C.

2) Mobile phase A (0.1% TFA in water):

Example of preparation: 1000 mL of purified water was measured out using a graduated cylinder, and 1 mL of TFA (sigma) was added. The solution was well mixed before use and was stored at 2-8 °C for 14 days.

3) Mobile phase B (0.1% TFA in acetonitrile):

Example of preparation: 1000 mL of acetonitrile was measured out using a graduated cylinder, and 1 mL of TFA was added. The solution was well mixed before use and was stored at 2-8 °C for 14 days.

3. Data analysis

Comparisons were made to the spectra of the sample and the naked antibody to identify the locations of the light chain and heavy chain, and then integration was performed on the spectrum of the test sample to calculate the DAR value.

The calculation formula is as follows:

| Name | Number of linked drugs |
|---|---|
| LC | 0 |
| LC+1 | 2 |
| HC | 0 |
| HC+1 | 2 |
| HC+2 | 4 |
| HC+3 | 6 |

$$\text{Total LC peak area} = \text{LC peak area} + \text{LC+1 peak area}$$

$$\text{Total HC peak area} = \text{HC peak area} + \text{HC+1 peak area} + \text{HC+2 peak area} + \text{HC+3 peak area}$$

$$\text{LC DAR} = \Sigma(\text{number of linked drugs} \times \text{percent peak area})/\text{total LC peak area}$$

$$\text{HC DAR} = \Sigma(\text{number of linked drugs} \times \text{percent peak area})/\text{total HC peak area}$$

$$\text{DAR} = \text{LC DAR} + \text{HC DAR}.$$

**Example 3: Preparation of ADC-1**

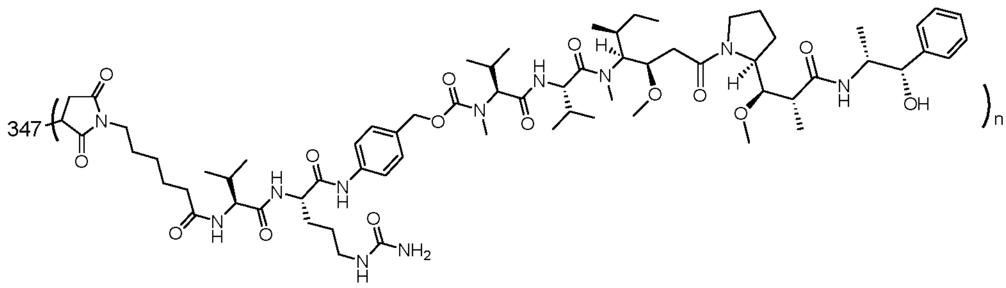

ADC-1

To an aqueous buffer solution of antibody 347 in PBS (0.05 M aqueous PBS buffer solution at pH 6.5; 10.0 mg/mL, 3.14 mL, 212 nmol) was added a prepared aqueous solution of tris(2-carboxyethyl)phosphine (TCEP) hydrochloride (10 mM, 55.2 μL, 552 nmol) at 37 °C. The reaction system was shaken on a water bath shaker at 37 °C for 3 h, and then the reaction was stopped. The reaction solution was cooled to 25 °C in a water bath.

Compound D1 (Biochempartner, CAS: 646502-53-6, 2.92 mg, 2218 nmol) was dissolved in 140 μL of DMSO, and the resulting solution was added to the above reaction solution. The mixture was shaken on a water bath shaker at 25 °C for 3 h, and then the reaction was stopped. The reaction solution was desalted and purified through a Sephadex G25 gel column (elution phase: 0.05 M aqueous PBS buffer solution at pH 6.5, containing 0.001 M EDTA) to give the title product ADC-1 in PBS buffer (2.25 mg/mL, 13.5 mL), which was refrigerated and stored at 4 °C.

Average drug loading calculated by RP-HPLC: n = 4.04.

**Example 4: Preparation of ADC-2**

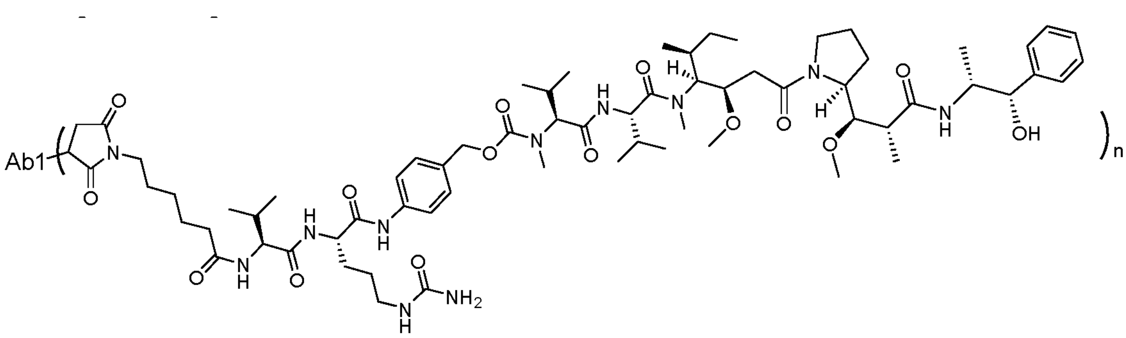

ADC-2

To an aqueous buffer solution of antibody Ab1 in PBS (0.05 M aqueous PBS buffer solution at pH 6.5; 10.0 mg/mL, 1.44 mL, 97.3 nmol) was added a prepared aqueous solution of tris(2-carboxyethyl)phosphine (TCEP) hydrochloride (10 mM, 25.3 μL, 253 nmol) at 37 °C. The reaction system was shaken on a water bath shaker at 37 °C for 3 h, and then the reaction was stopped. The reaction solution was cooled to 25 °C in a water bath.

Compound D1 (1.28 mg, 972 nmol) was dissolved in 65 μL of DMSO, and the resulting solution was added to the above reaction solution. The mixture was shaken on a water bath shaker at 25 °C for 3 h, and then the reaction was stopped. The reaction solution was desalted and purified through a Sephadex G25 gel column (elution phase: 0.05 M aqueous PBS buffer solution at pH 6.5, containing 0.001 M EDTA) to give the title product ADC-2 in PBS buffer (1.24 mg/mL, 11 mL), which was refrigerated and stored at 4 °C.

Average drug loading calculated by RP-HPLC: n = 4.43.

**Example 5: Preparation of ADC-3**

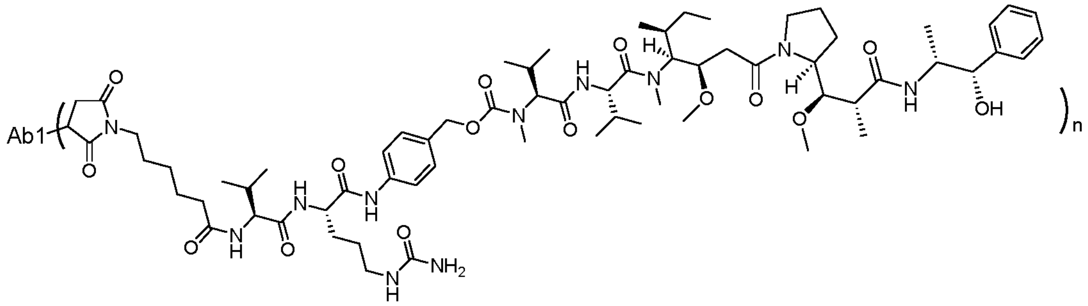

ADC-3

To an aqueous buffer solution of antibody Ab1 in PBS (0.05 M aqueous PBS buffer solution at pH 6.5; 10.0 mg/mL, 6 mL, 405 nmol) was added a prepared aqueous solution of tris(2-carboxyethyl)phosphine (TCEP) hydrochloride (10 mM, 101.4 μL, 1014 nmol) at 37 °C. The reaction system was shaken on a water bath shaker at 37 °C for 3 h, and then the reaction was stopped. The reaction solution was cooled to 25 °C in a water bath.

Compound D1 (5.34 mg, 4058 nmol) was dissolved in 300 μL of DMSO, and the resulting solution was added to the above reaction solution. The mixture was shaken on a water bath shaker at 25 °C for 3 h, and then the reaction was stopped. The reaction solution was desalted and purified through a Sephadex G25 gel column (elution phase: 0.05 *M* aqueous PBS buffer solution at pH 6.5, containing 0.001 M EDTA) to give the title product **ADC-3** in PBS buffer (3.33 mg/mL, 18 mL), which was refrigerated and stored at 4 °C.

Average drug loading calculated by RP-HPLC: n = 4.76.

**Example 6: Preparation of ADC-4**

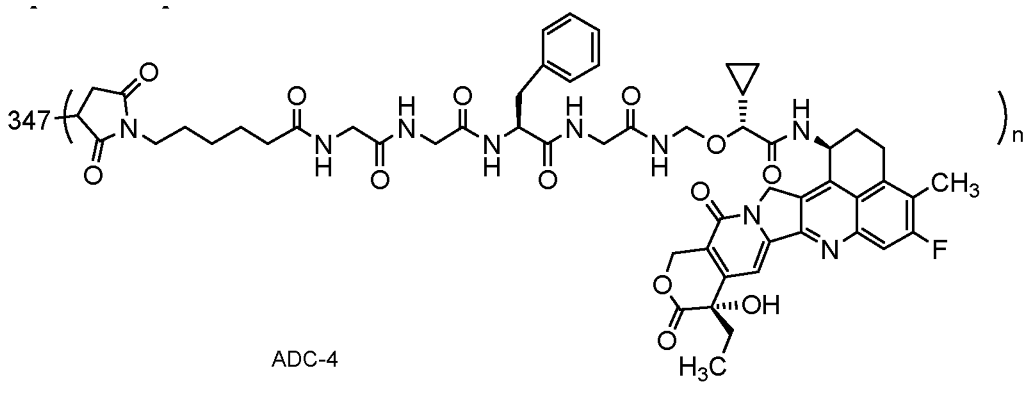

ADC-4

To an aqueous buffer solution of antibody **347** in PBS (0.05 M aqueous PBS buffer solution at pH 6.5; 10.0 mg/mL, 30 mL, 2027 nmol) was added a prepared aqueous solution of tris(2-carboxyethyl)phosphine (TCEP) hydrochloride (10 mM, 1.145 mL, 11.45 μmol) at 37 °C. The reaction system was shaken on a water bath shaker at 37 °C for 3.5 h, and then the reaction was stopped. The reaction solution was cooled to 25 °C in a water bath.

**9A** (prepared according to the method for compound 9-A of Example 9 in WO2020063676, which is incorporated in the present disclosure in its entirety) (32.7 mg, 30.45 μmol) was dissolved in 1.7 mL of dimethyl sulfoxide, and the resulting solution was added dropwise to the above reaction solution. The reaction system was shaken on a water bath shaker at 25 °C for 3 h, and then the reaction was stopped. The reaction solution was desalted and purified through a Sephadex G25 gel column (elution phase: 0.05 M aqueous PBS buffer solution at pH 6.5, containing 0.001 M EDTA) to give the title product **ADC-4** in PBS buffer (2.6 mg/mL, 80 mL), which was refrigerated and stored at 4 °C.

Average drug loading calculated by RP-HPLC: n = 7.39.

**Example 7: Preparation of ADC-5**

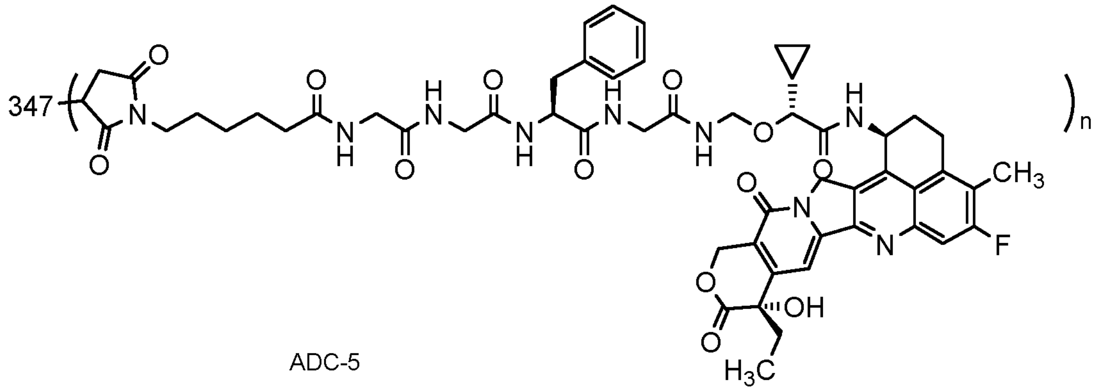

ADC-5

To an aqueous buffer solution of antibody **347** in PBS (0.05 M aqueous PBS buffer solution at pH 6.5; 10.0 mg/mL, 10 mL, 675 nmol) was added a prepared aqueous solution of tris(2-carboxyethyl)phosphine hydrochloride (TCEP.HCl) (10 mM, 169 μL, 1690 nmol) at 37 °C. The reaction system was shaken on a water bath shaker at 37 °C for 3.5 h, and then the reaction was stopped. The reaction solution was cooled to 25 °C in a water bath.

Compound **9A** (7.26 mg, 6.76 μmol) was dissolved in 500 μL of dimethyl sulfoxide, and the resulting solution was added dropwise to the above reaction solution. The reaction system was shaken on a water bath shaker at 25 °C for 3 h, and then the reaction was stopped. The reaction solution was desalted and purified through a Sephadex G25 gel column (elution phase: 0.05 M aqueous PBS buffer solution at pH 6.5, containing 0.001 M EDTA) to give the title product **ADC-5** in PBS buffer (1.92 mg/mL, 48 mL), which was stored at 4 °C.

Average drug loading calculated by RP-HPLC: n = 3.99.

**Test Examples**

**Biological Evaluations**

**Test Example 1: Antibody Protein Level Affinity and Kinetics**

The anti-ROR1 antibody was analyzed by Biacore T200 (Cytiva) for affinity characterization and binding kinetics. The IgG antibody was subjected to affinity capture by a Protein A biosensor chip (Cat. # 29127556, Cytiva), and then a human ROR1 (Cat. # 19771-H08H, Sino Biological) antigen that was diluted with the HBS-EP buffer (pH 7.4, Cat. # BR-1006-69, Cytiva) to a series of concentrations was allowed to flow over the surface of the chip. The antigen-antibody binding kinetics was tracked for 3 min and the dissociation kinetics was tracked for 10 min. Reaction signals were detected in real time using a Biacore T200 instrument to obtain binding and dissociation curves. After dissociation was complete in each cycle, the biosensor chip was washed with 10 mM Gly-HCl at pH 1.5 (Cat. # BR-1003-54, Cytiva) for regeneration. The data obtained were analyzed with BIAevaluation software of Cytiva using a 1:1 (Langmuir) binding model. The ka (kon), kd (koff) and KD values determined in this way are shown in the table below.

Table 3. Affinity

| Antibody | Immo level/RU | ka (1/Ms) | kd (1/s) | KD (M) |
|---|---|---|---|---|
| Ab1 | 175 | 6.00E+05 | 5.46E-02 | 9.10E-08 |
| 347 | 175 | 2.31E+06 | 2.73E-03 | 1.18E-09 |

**Test Example 2: *In Vitro* Binding of Antibody to Cells**

ROR1-CHO-K1 cells (Example 1), Jeko-1 cells (ATCC, CRL-3006), or MDA-MB-231 cells (ATCC, CRM-HTB-26) were separately suspended in a FACS buffer (2% fetal bovine serum (Gibco, 10099141) and PBS at pH 7.4 (Sigma, P4417-100TAB) to prepare a cell suspension at 1 × $10^6$ cells/mL, and the suspension was then added to a 96-well round-bottom plate at 100 μL/well. After centrifugation and removal of the supernatant, the test antibody that was diluted

with the FACS buffer to different concentrations was added at 50 μL/well. The plate was incubated in the dark in a 4 °C refrigerator for 1 h. The plate was washed 3 times with the FACS buffer by centrifugation at 500 g, and a fluorescent secondary antibody at a working concentration was added. The plate was incubated in the dark in the 4 °C refrigerator for 40 min. The plate was washed 3 times with the FACS buffer by centrifugation at 500 g and tested on a BD FACSCantoII flow cytometer for geometric mean fluorescence intensity, and the $EC_{50}$ values for the binding of the antibody to the cells expressing ROR1 were calculated.

Table 4. Binding activity of antibody to cells

| | ROR1-CHO-K1 cells | Jeko-1 cells | MDA-MB-231 cells |
|---|---|---|---|
| Antibody | 347 | 347 | 347 |
| Maximal fluorescence value | 19611 | 3554 | 2739 |
| $EC_{50}$ (nM) | 0.502 | 0.098 | 0.094 |

**Test Example 3: DT3C Antibody Endocytosis Assay**

The purpose of this assay is that the activated diphtheria toxin (DT) kills cells after the DT3C protein enters the cells, indirectly reflecting endocytosis of the ROR1 antibody. The *in vitro* endocytic activity of the anti-ROR1 antibody was evaluated according to $IC_{50}$ and maximal killing values.

DT3C is a recombinantly expressed fusion protein formed by fusing fragment A of diphtheria toxin (toxin portion only) and fragment 3C of group G streptococcus (IgG binding portion). The protein has a high affinity for the IgG portion of an antibody. It enters cells together with the IgG portion when the antibody is endocytosed, and releases toxic DT under the action of intracellular furin protease. The DT can inhibit the activity of EF2-ADP ribosylation, block the protein translation process and finally cause cell death. DT3C that does not enter the cell has no activity of cell killing. The endocytic activity of the antibody was evaluated according to cell killing.

The ROR1-CHOK1 cell suspension was prepared using a fresh cell medium containing 20% low IgG FBS at a cell density of $2 \times 10^4$ cells/mL, and added into the cell culture plate at 50 μL/well. The plate was incubated with 5% carbon dioxide at 37 °C for 16 h. DT3C was diluted to 1.6 μM with a serum-free medium, and the anti-ROR1 antibody was diluted to 266.4 nM with the serum-free medium. 80 μL of DT3C and 80 μL of the anti-ROR1 antibody were mixed (1:1, v/v) and incubated at room temperature for 30 min. The molar concentration of DT3C was 6 times that of the anti-ROR1 antibody. The mixture of DT3C and the anti-ROR1 antibody was serially diluted 4-fold with the serum-free medium to 8 concentrations. The 9th and 10th points were pure media. 50 μL of the diluted mixture was added to 50 μL of cells and incubated in an incubator for three days. To each well, 50 μL of CTG was added. The plate was incubated in the dark at room temperature for 10 min. A white membrane was attached to the bottom of the cell culture plate. The plate was placed on a microplate reader Victor 3, and chemiluminescence readings were taken.

Table 5. Results of DT3C endocytosis killing experiments on ROR1-CHOK1

| Antibody | 347 |
|---|---|
| Maximal killing value | 87.39% |
| $IC_{50}$ (nM) | 0.596 |

**Test Example 4: Cross-Binding Ability to Rat/Mouse ROR1 antigen**

The anti-ROR1 antibody was analyzed by Biacore T200 (Cytiva) for affinity characterization and binding kinetics. The IgG antibody was subjected to affinity capture by a Protein A biosensor chip (Cat. # 29127556, Cytiva), and then a mouse ROR1-His (Cat. # RO1-M522, Aero) antigen and a rat ROR1-His (Cat. # RO1-R5221, Aero) antigen that were diluted with the HBS-EP buffer (pH 7.4, Cat. # BR-1006-69, Cytiva) to a series of concentrations were allowed to flow over the surface of the chip. The antigen-antibody binding kinetics was tracked for 3 min and the dissociation kinetics was tracked for 15 min. Reaction signals were detected in real time using a Biacore T200 instrument to obtain binding and dissociation curves. After dissociation was complete in each cycle, the biosensor chip was washed with 10 mM Gly-HCl at pH 1.5 (Cat. # BR-1003-54, Cytiva) for regeneration. The data obtained were analyzed with BIAevaluation software of Cytiva using a 1:1 (Langmuir) binding model. The ka (kon), kd (koff) and KD values determined in this way are shown in the table below.

Table 6: Binding ability of 347 and Ab 1 to rat/mouse ROR1 antigen

| Antibody | Antigen | KD (M) | ka (1/Ms) | kdis (1/s) |
|---|---|---|---|---|
| 347 | Mouse ROR1 | 2.38E-10 | 2.84E+06 | 6.76E-04 |
| | Rat ROR1 | 3.15E-11 | 3.66E+07 | 1.15E-03 |
| Ab1 | Mouse ROR1 | No binding | | |
| | Rat ROR1 | | | |

Conclusion:

The 347 antibody has relatively cross-binding to rat/mouse ROR1.

**Test Example 5: *In Vivo* Efficacy Evaluation of ADC Molecules in High-Expression CDX Model**

NDG mice were inoculated subcutaneously on the right flank with JEKO-1 cells (human mantle cell lymphoma, MCL, $5 \times 10^6$ cells + 50% matrigel/mouse/200 μL). After 8 days, the mice were grouped, 8 mice/group. The mean tumor volume per group on the day of grouping was 223.09 $mm^3$. The ADC compound was administered intraperitoneally at a dose of 5 mpk once a week for 1 administration in total, and the mice were observed for 21 days.

The tumor volumes and body weights were measured twice a week, and the data were recorded. Excel 2003 statistical software was used. The mean values were calculated as avg; the SD values were calculated as STDEV; the SEM values were calculated as STDEV/SQRT; and the inter-group difference P-value was calculated as TTEST.

Tumor volume (V) was calculated using the formula: $V = 1/2 \times L_{long} \times L_{short}^2$

Relative tumor proliferation rate $T/C\% = (T - T0)/(C - C0) \times 100\%$

Tumor growth inhibition TGI (%) = 1 - T/C (%),

wherein T and C are the tumor volumes of animals at the end of the experiment in the treatment group and control group, respectively; T0 and C0 are the tumor volumes of animals at the beginning of the experiment.

Table 7. Efficacy of ADCs against Jeko-1 xenograft tumors in tumor-bearing nude mice

| Antibody | Tumor growth inhibition TGI (%) |
|---|---|
| ADC-2 5mpk | 85.6 |
| ADC-1 5mpk | 93.4 |

Conclusion:

In the experiment, the tumor growth inhibition of the test ADC-1 (5 mpk) reached 93.4% ($P < 0.0001$ vs blank control); the tumor growth inhibition of the positive control ADC-2 (5 mpk), was 85.6% ($p < 0.0001$ vs blank control). In terms of the tumor growth inhibition, the tumor growth inhibition of ADC-1 (5 mpk) is better than that of the positive control ADC-2 (5 mpk).

**Test Example 6: Cell Activity Evaluation of ADC Molecules**

The purpose of this assay is to examine the killing effects of ROR1-ADC samples, ADC-4 and ADC-5, on cells and to evaluate the *in vitro* activity of ROR1-ADC according to $IC_{50}$ and maximal killing values.

HCC827/ROR1 (the cell line overexpressing ROR1), Jeko-1, MDA-MB-231, and CHO-K1 cells were digested with pancreatin, neutralized with a fresh medium, centrifuged at 1000 rpm, and then resuspended in a medium. After the cells were counted, the cell suspension was adjusted to an appropriate density and added to a 96-well cell culture plate at 135 μL/well. Only 150 μL of the medium was added to the remaining peripheral wells of the plate. The culture plate was incubated in an incubator at 37 °C with 5% carbon dioxide for 24 h.

The ADC samples were formulated into different concentrations with PBS and diluted five-fold with PBS to 8 concentrations. The cell culture plate was taken out, and 15 μL of the 10× solution was added to each well. The plate was cultured at 37 °C with 5% carbon dioxide for 6 days.

To each well, 70 μL of CTG was added. The plate was incubated at room temperature in the dark for 10 min. A white membrane was attached to the bottom of the cell culture plate, and the chemiluminescence readings were taken on Victor3. The data from this assay were processed using the data processing software GraphPad prism 5.0.

Table 8. Killing effects of test drugs on various cell lines

| ADC Sample | HCC827/ROR1 (+++) | | Jeko-1 (+) | | MDA-MB-231 (+) | | CHO-K1 (-) | |
|---|---|---|---|---|---|---|---|---|
| | $IC_{50}$ (nM) | IMax% | $IC_{50}$ (nM) | IMax% | $IC_{50}$ (nM) | IMax% | $IC_{50}$ (nM) | IMax% |
| ADC-4 | 1.36 | 94.83 | 17.36 | 99.96 | 146.30 | 91.71 | >1100 | 36.40 |
| ADC-5 | 4.36 | 91.42 | 58.10 | 99.93 | 348.80 | 87.28 | >1100 | 54.66 |
| Note: "+++" indicates high expression of ROR1, "+" indicates low expression of ROR1, and "-" indicates no expression of ROR1. | | | | | | | | |

Conclusion: The inhibitory activity of ADC-4 and ADC-5 to different cell lines is in positive correlation with the antigen expression level, that is, the higher the antigen expression level is, the stronger the inhibitory activity of the drug is.

**Test Example 7: *In Vivo* Activity Evaluation of ADC Molecules in CDX Model**

**1. Human mantle cell lymphoma (MCL) mouse subcutaneous xenograft tumor model**

NDG mice were inoculated subcutaneously on the right flank with JEKO-1 cells ($5 \times 10^6$ cells + 50% matrigel/mouse/200 μL/mouse). After 7 days, the mice were grouped, 8 mice/group. The mean tumor volume per group on the day of grouping was 266.7 $mm^3$. The ADC compound was administered intraperitoneally at a dose of 2.5 mpk/5 mpk on day 1 and day 14 after grouping, respectively, for 2 administrations in total, and the mice were observed for 21 days.

The tumor volumes and body weights were measured twice a week, and the data were recorded. Excel 2003 statistical software was used. The mean values were calculated as avg; the SD values were calculated as STDEV; the SEM values were calculated as STDEV/SQRT; and the inter-group difference P-value was calculated as TTEST.

Tumor volume (V) was calculated using the formula: $V = 1/2 \times L_{long} \times L_{short}^2$
Relative tumor proliferation rate T/C% = $(T - T0)/(C - C0) \times 100\%$
Tumor growth inhibition TGI (%) = 1 - T/C (%),
wherein T and C are the tumor volumes of animals at the end of the experiment in the treatment group and control group, respectively; T0 and C0 are the tumor volumes of animals at the beginning of the experiment.

Table 9. Efficacy of test drugs against Jeko-1 xenograft tumors in tumor-bearing NDG mice (D21)

| Test drug | Dose | Tumor growth inhibition TGI (%) |
|---|---|---|
| ADC-4 | 2.5mpk | 86.28 |
| ADC-4 | 5mpk | 100 |
| ADC-5 | 2.5mpk | 45.03 |
| ADC-5 | 5mpk | 63.60 |

Conclusion:

In the Jeko-1 mouse subcutaneous xenograft tumor CDX model, the tumor inhibition activity of ADC-4 is better than that of ADC-5, and the tumor growth inhibition of the test ADC-4 (5 mpk) reaches 100% ($P < 0.0001$ vs blank control).

**2. Triple-negative breast cancer (TNBC) mouse subcutaneous xenograft tumor model**

60 NOD-SCID mice were inoculated subcutaneously on the right flank with MDA-MB-231 cells ($3 \times 10^6$ cells/200 μL/mouse). After 9 days, the mice were grouped, 7 mice/group. The mean tumor volume per group on the day of grouping was 184.85 $mm^3$. The ADC compound was administered intraperitoneally at a dose of 2.5 mpk/5 mpk only on day 1 after grouping, and the mice were observed for 21 days.

The tumor volumes and body weights were measured twice a week, and the data were recorded. Excel 2003 statistical software was used. The mean values were calculated as avg; the SD values were calculated as STDEV; the SEM values were calculated as STDEV/SQRT; and the inter-group difference P-value was calculated as TTEST.

Tumor volume (V) was calculated using the formula: $V = 1/2 \times L_{long} \times L_{short}^2$
Relative tumor proliferation rate T/C% = (T - T0)/(C - C0) × 100%
Tumor growth inhibition TGI (%) = 1 - T/C (%),
wherein T and C are the tumor volumes of animals at the end of the experiment in the treatment group and control group, respectively; T0 and C0 are the tumor volumes of animals at the beginning of the experiment.

Table 10. Efficacy of test drugs against MDA-MB-231 xenograft tumors in tumor-bearing NOD-SCID mice (D20)

| Test drug | Dose | Tumor growth inhibition TGI (%) |
|---|---|---|
| ADC-4 | 2.5mpk | 74.53 |
| ADC-4 | 5mpk | 100 |
| ADC-3 | 5mpk | 75.79 |
| ADC-5 | 2.5mpk | 77.31 |
| ADC-5 | 5mpk | 92.21 |

Conclusion:

In the MDA-MB-231 mouse subcutaneous xenograft tumor CDX model, after the mice were observed for 20 days, the tumor growth inhibition of the test ADC-4 (5 mpk) reached 100% (P < 0.0001 vs blank control); the tumor growth inhibition of positive ADC-3 (5 mpk) was 75.79% (p < 0.0001 vs blank control); the tumor inhibition of the test ADC-5 (5 mpk) reached 92.21% (p < 0.0001 vs blank control). In terms of the tumor growth inhibition, the tumor growth inhibition of ADC-4 (5 mpk) is better than that of positive ADC-3 (5 mpk) and ADC-5 (5 mpk), and no significant toxicity is exhibited in terms of body weight changes.

**Test Example 8: PK Evaluation of ADC molecule**

Single-dose pharmacokinetics of ROR1-ADC (ADC-4) were studied in adult SD rats (Zhejiang Vital River Laboratory Animal Technology Co., Ltd.) and NDG C57 mice (Balbc). Rats were randomly divided into one group, and the ROR1-ADC molecule was injected intravenously at 10 mpk (n = 4 mouse/group). Mice were randomly divided into two groups, and the ROR1-ADC molecule was injected intravenously at 10 mpk (n = 3 mouse/group). Sera were collected at 0.083 h, 8 h, 24 h, 48 h, 96 h, 168 h, 240 h, 336 h, 504 h, and 672 h after intravenous injection in the rats for bioanalytical measurements; sera were collected at 0.083 h, 8 h, 48 h, 168 h, 336 h, and 672 h after intravenous injection in one group of the mice for bioanalytical measurements; sera were collected at 1 h, 24 h, 96 h, 240 h, and 504 h after intravenous injection in the other group of the mice for bioanalytical measurements. The rat/mouse serum samples were determined using an HTRF method, and the quantitative analysis of the content of the test samples was performed by a four-parameter model curve of the standard substance.
Pharmacokinetic parameters were analyzed using a standard non-compartmental model of WinNonlin software (6.4).
The experimental results are as follows:

Table 11. Pharmacokinetic parameters of test drug in rats or mice

| ADC-4 10mpk | Rat | | Mice | |
|---|---|---|---|---|
| | Total antibody | Intact ADC | Total antibody | Intact ADC |
| T1/2 (days) | 6.5 ± 1.1 | 5.7 ± 1.1 | 1.3 ± 0.3 | 1.4 ± 0.3 |
| AUC 0-t (ug/ml*h) | 11783 | 10731 | 5157 | 4869 |
| Cmax (ug/ml) | 198.17 | 192.73 | 187.47 | 180.91 |
| CL (mL/day/kg) | 13.89 | 16.43 | 46.06 | 48.50 |

(continued)

| ADC-4 10mpk | Rat | | Mice | |
|---|---|---|---|---|
| | Total antibody | Intact ADC | Total antibody | Intact ADC |
| AUC intact ADC/total antibody | 76% | | 94% | |

Conclusion:

In rats and mice, ADC-4 has relatively good plasma stability.

**Test Example 9: Toxicity Evaluation of ADC Molecules in Rats**

SD rats (male, Zhejiang Vital River Laboratory Animal Technology Co., Ltd.) were injected with the ADC compound (prepared in normal saline) through the tail vein, with 6 rats in each group. The rats were administered once a week, for 2 administrations in total, and each rat was injected at 5 mL/kg according to the body weight. The specific experimental design and experimental results are as follows:

Table 12. Administration regimen for toxicity tests

| Group | ADC compound | Administration dose (mg/kg) |
|---|---|---|
| Vehicle | Normal saline | 0 |
| A | ADC-4 | 35 |
| B | ADC-4 | 70 |
| C | ADC-3 | 35 |

The results show that rats died after a single administration of the positive control ADC-3, and there was no rat death after two administrations of ADC-4 at doses of 35 mpk & 70 mpk, respectively. During the administration period, animals in group C (ADC-3-35mpk) showed severe hunched postures, piloerection, thinness, and significant clinical adverse reactions such as significant hind limb hypodynamia, bradykinesia, hind limb paralysis and the like, and normal food and water intake was influenced after the first administration; some animals had died. To prevent the data from being censored, the remaining animals in group C were dissected in advance on day 6 after administration. No significant abnormality was observed in animals of groups A and B after administration.

Table 13. Toxicity experiment results for ADC compounds in SD rats

| Indicator | ADC-3-35mpk Single administration | ADC-4-35mpk Two administrations | ADC-4-70mpk Two administrations |
|---|---|---|---|
| Number of dead animals/total number of animals | 6 rats/6 rats | 0 rat/6 rats | 0 rat/6 rats |
| Clinical observations | On day 3 to day 6 after single administration, rats showed hunched postures and piloerection, extreme thinness, and hind limb paralysis, and food and water intake was normal | After single administration or two administrations, significant infusion reaction was found | After single administration or two administrations, significant infusion reaction was found |
| Body weight | Day 3: ♂: ↓37%**, ♀: ↓18%** | Day 10: ♂: ↓9%** ♀: ↓9% | Day 10: ♂: ↓19%** ♀: ↓6% |

(continued)

| Indicator | ADC-3-35mpk Single administration | ADC-4-35mpk Two administrations | ADC-4-70mpk Two administrations |
|---|---|---|---|
| Hematology | WBC: ♂↓57%, ♀↓87.5%<br>PLT: ♂↓98.5%, ♀↓89.4%<br>RET#: ♂↓94.9%, ♀↓93.3% | There was no significant change in WBC, PLT: ♂: ↑25%*, ♀↑46%<br>RET# ♂: ↑35%*, ♀↑56%* | WBC: ♂↓49% *, ♀↓49%*<br>PLT: ♂↑32%*, ♀↑70%<br>RET# ♂↑56%*, ♀↑79%* |
| Blood biochemistry | ALT ♂: ↑254%* | Normal | ALT ♂: ↑42%*, ♀: ↑58% |
| Gross pathology and viscera/body ratio | Thymus ♂: ↓68%*; ♀: ↓52%<br>Organ coefficients of liver, lung, and kidney↑ | Thymus ♂↓70%*, ♀↓: 77%* | Thymus ♂↓74%*, ♀↓77%* |
| Note: WBC (white blood cell), PLT (platelet), RET# (reticulocyte), and ALT (glycine aminotransferases). | | | |

Conclusion:

ADC-4-35/70mpk: maximum tolerated dose (MTD) > 70 mpk; toxicity performance: slow weight gain, thymus atrophy, and the like;

ADC-3-35mpk: MTD < 35 mpk (death of the animal occurred); toxicity performance: weight loss, bone marrow suppression, significant thymus atrophy, and significant influence on the functions of organs such as liver, lung, kidney, and the like.

The experimental results suggest that ADC-4 has better *in vivo* safety and lower toxic and side effects compared to the control molecule ADC-3.

## Claims

**1.** An isolated anti-ROR1 antibody, comprising:

a HCDR1, a HCDR2, and a HCDR3 comprised in a heavy chain variable region set forth in SEQ ID NO: 2; and a LCDR1, a LCDR2, and a LCDR3 comprised in a light chain variable region set forth in SEQ ID NO: 3.

**2.** An isolated anti-ROR1 antibody, comprising a heavy chain variable region and a light chain variable region, wherein:

the heavy chain variable region comprises a HCDR1, a HCDR2, and a HCDR3 set forth in SEQ ID NO: 4, SEQ ID NO: 5, and SEQ ID NO: 6, respectively;
the light chain variable region comprises a LCDR1, a LCDR2, and a LCDR3 set forth in SEQ ID NO: 7, SEQ ID NO: 8, and SEQ ID NO: 9, respectively.

**3.** The isolated anti-ROR1 antibody according to claim 1 or 2, comprising a heavy chain variable region and a light chain variable region, wherein:

the heavy chain variable region comprises an amino acid sequence having at least 90% sequence identity to SEQ ID NO: 2, and/or the light chain variable region comprises an amino acid sequence having at least 90% sequence identity to SEQ ID NO: 3;
preferably,

the amino acid sequence of the heavy chain variable region is set forth in SEQ ID NO: 2; and
the amino acid sequence of the light chain variable region is set forth in SEQ ID NO: 3.

**4.** The isolated anti-ROR1 antibody according to any one of claims 1 to 3, comprising:

a heavy chain having at least 85% sequence identity to SEQ ID NO: 12, and/or
a light chain having at least 85% sequence identity to SEQ ID NO: 13;
wherein preferably, the isolated anti-ROR1 antibody comprises:
a heavy chain set forth in SEQ ID NO: 12 and a light chain set forth in SEQ ID NO: 13.

**5.** The isolated anti-ROR1 antibody according to any one of claims 1 to 4, binding to human ROR1 or an epitope thereof with a KD of less than $1 \times 10^{-8}$ M, wherein the KD is measured by surface plasmon resonance.

**6.** An isolated nucleic acid molecule encoding the isolated anti-ROR1 antibody according to any one of claims 1 to 5.

**7.** A host cell comprising the isolated nucleic acid molecule according to claim 6.

**8.** A method for immunodetection or determination of ROR1, comprising a step of contacting a subject or a sample from the subject using the isolated anti-ROR1 antibody according to any one of claims 1 to 5.

**9.** An immunoconjugate or a pharmaceutically acceptable salt thereof, comprising:

the isolated anti-ROR1 antibody according to any one of claims 1 to 5 and an effector, wherein the effector is conjugated to the anti-ROR1 antibody;
preferably, the effector is selected from the group consisting of: a radioisotope, an anti-tumor agent, an immunomodulator, a biological response modifier, a lectin, a cytotoxic drug, a chromophore, a fluorophore, a chemiluminescent compound, an enzyme, a metal ion, and any combination thereof.

**10.** The immunoconjugate or the pharmaceutically acceptable salt thereof according to claim 9, wherein, the structure of the immunoconjugate is selected from the group consisting of:

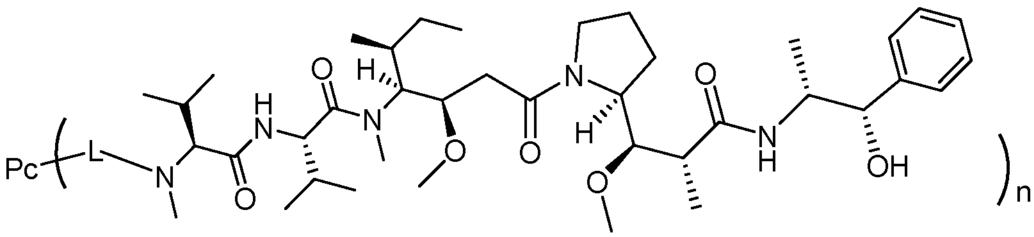

and

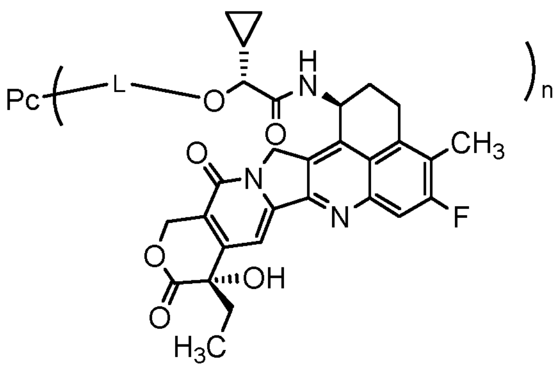

wherein:

n is an integer or a decimal from 1 to 10, preferably an integer or a decimal from 1 to 8, and more preferably 8;

L is a linker unit, and Pc is the anti-ROR1 antibody according to any one of claims 1 to 5.

**11.** The immunoconjugate or the pharmaceutically acceptable salt thereof according to claim 10, wherein linker unit -L- is $-L^1-L^2-L^3-L^4-$, wherein

$L^1$ is selected from the group consisting of -(succinimidin-3-yl-*N*)-W-C(O)-, $-CH_2-C(O)-NR^1-W-C(O)-$, or -C(O)-W-C(O)-, wherein W is selected from the group consisting of $C_{1-8}$ alkyl or $C_{1-6}$ alkyl-cycloalkyl;
$L^2$ is selected from the group consisting of $-NR^2(CH_2CH_2O)p^1CH_2CH_2C(O)-$, $-NR^2(CH_2CH_2O)p^1CH_2C(O)-$, $-S(CH_2)p^1C(O)-$, or a chemical bond, wherein $p^1$ is an integer from 1 to 20; preferably, a chemical bond;
$L^3$ is a peptide residue consisting of 2 to 7 amino acid residues, wherein the amino acid residues are selected from the group consisting of amino acid residues formed from phenylalanine, glycine, valine, lysine, citrulline, serine, glutamic acid, and aspartic acid; $L^4$ is selected from the group consisting of $-NR^3(CR^4R^5)t-$, $-C(O)NR^3-$, $-C(O)NR^3(CH_2)_t-$, or a PAB group, wherein t is an integer from 1 to 6;
$R^1$, $R^2$, and $R^3$ are identical or different and are each independently selected from the group consisting of a hydrogen atom, alkyl, haloalkyl, deuterated alkyl, and hydroxyalkyl;
$R^4$ and $R^5$ are identical or different and are each independently selected from the group consisting of a hydrogen atom, halogen, alkyl, haloalkyl, deuterated alkyl, and hydroxyalkyl.

**12.** The immunoconjugate or the pharmaceutically acceptable salt thereof according to claim 10, wherein the structure of the immunoconjugate is selected from the group consisting of:

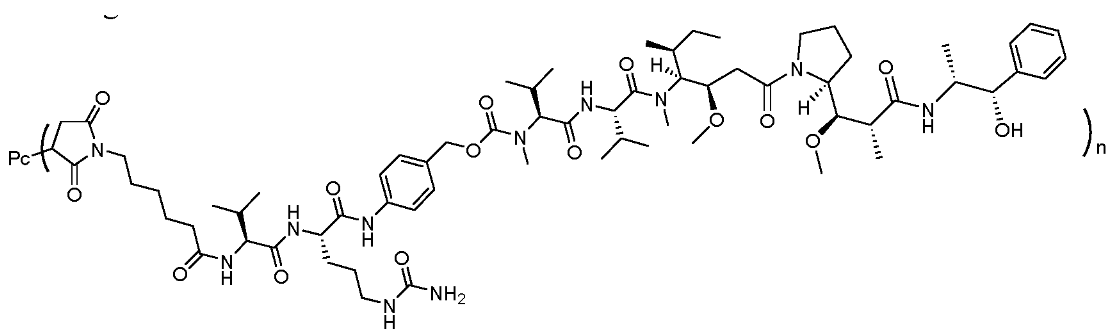

and

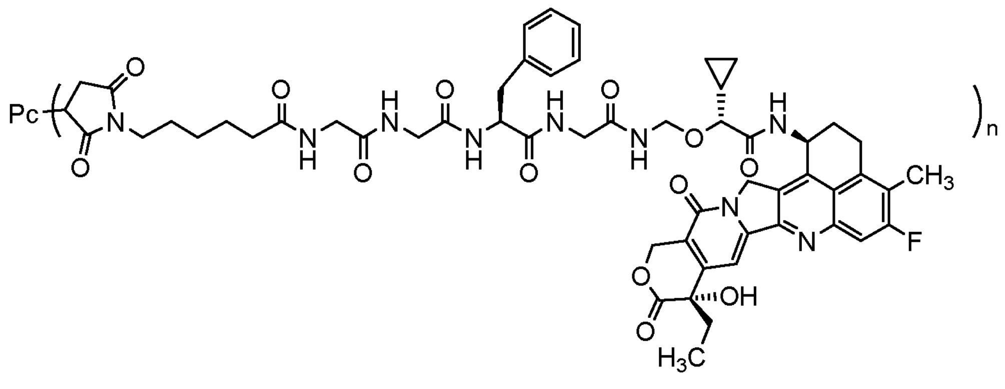

wherein:
n and Pc are as defined in claim 10.

**13.** A pharmaceutical composition, comprising:

the isolated anti-ROR1 antibody according to any one of claims 1 to 5, the isolated nucleic acid molecule according to claim 6, or the immunoconjugate or the pharmaceutically acceptable salt thereof according to any one of claims 9 to 12, and
one or more pharmaceutically acceptable excipients, diluents, or carriers.

14. Use of the isolated anti-ROR1 antibody according to any one of claims 1 to 5, or the isolated nucleic acid molecule according to claim 6, or the immunoconjugate or the pharmaceutically acceptable salt thereof according to any one of claims 9 to 12, or the pharmaceutical composition according to claim 13 in the preparation of a drug for treating a ROR1-mediated disease or disorder.

15. Use of the isolated anti-ROR1 antibody according to any one of claims 1 to 5, or the isolated nucleic acid molecule according to claim 6, or the immunoconjugate or the pharmaceutically acceptable salt thereof according to any one of claims 9 to 12, or the pharmaceutical composition according to claim 13 in the preparation of a drug for treating a tumor or cancer, wherein
preferably, the tumor or cancer is selected from the group consisting of breast cancer, pancreatic cancer, lung cancer, esophageal cancer, non-small cell lung cancer, laryngeal tumors, sarcoma, pharyngeal tumors, oral tumors, gastric cancer, ovarian cancer, prostate cancer, bladder cancer, colorectal cancer, lymphoma and leukemia.

**INTERNATIONAL SEARCH REPORT**

International application No.
**PCT/CN2022/142644**

**A. CLASSIFICATION OF SUBJECT MATTER**

C07K 16/28(2006.01)i;C12N 15/13(2006.01)i;A61P35/00(2006.01)i

According to International Patent Classification (IPC) or to both national classification and IPC

**B. FIELDS SEARCHED**

Minimum documentation searched (classification system followed by classification symbols)

IPC：C07K；C12N；A61P

Documentation searched other than minimum documentation to the extent that such documents are included in the fields searched

Electronic data base consulted during the international search (name of data base and, where practicable, search terms used)

CNKI, CNABS, CNTXT, DWPI, SIPOABS, EPTXT, WOTXT, USTXT, ELSEVIER, ISI Web of Knowledge, PubMed, 中国专利生物序列检索系统, Chinese Patent Biological Sequence Retrieval System, NCBI, STN: ROR1, 抗体, antibody, 受体酪氨酸激酶样孤儿受体1, receptor tyrosine kinase like orphan receptor 1, 偶联物, 646502-53-6, SEQ ID NOs: 2-9, 12-13

**C. DOCUMENTS CONSIDERED TO BE RELEVANT**

| Category* | Citation of document, with indication, where appropriate, of the relevant passages | Relevant to claim No. |
|---|---|---|
| A | CN 107428833 A (JUNO THERAPEUTICS INC.) 01 December 2017 (2017-12-01)<br>description, abstract, and claims 1-119 | 1-15 |
| A | WO 2021057822 A1 (IMMUTHER PHARMTECH (SHANGHAI) CO., LTD.) 01 April 2021 (2021-04-01)<br>entire document | 1-15 |
| A | CN 113164621 A (HARBOUR BIOMED (SHANGHAI) CO., LTD.) 23 July 2021 (2021-07-23)<br>entire document | 1-15 |
| A | WO 2020063676 A1 (JIANGSU HENGRUI MEDICINE CO., LTD. et al.) 02 April 2020 (2020-04-02)<br>entire document | 1-15 |
| A | CN 110891972 A (UCL BUSINESS PLC) 17 March 2020 (2020-03-17)<br>entire document | 1-15 |
| A | US 2020354448 A1 (UCL BUSINESS LTD.) 12 November 2020 (2020-11-12)<br>entire document | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 March 2023** | **16 March 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/142644**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2022/142644**

| Patent document cited in search report | Publication date (day/month/year) | Patent family member(s) | Publication date (day/month/year) |
|---|---|---|---|
| CN 107428833 A | 01 December 2017 | TW 201639880 A | 16 November 2016 |
| | | TWI 718118 B | 11 February 2021 |
| | | EP 3760644 A1 | 06 January 2021 |
| | | EP 3245231 A1 | 22 November 2017 |
| | | EP 3245231 B1 | 12 August 2020 |
| | | ES 2818103 T3 | 09 April 2021 |
| | | US 2018265593 A1 | 20 September 2018 |
| | | US 10889652 B2 | 12 January 2021 |
| | | MX 2017009254 A | 12 October 2017 |
| | | AU 2016206457 A1 | 03 August 2017 |
| | | AU 2016206457 B2 | 11 November 2021 |
| | | JP 2021061857 A | 22 April 2021 |
| | | TW 202126682 A | 16 July 2021 |
| | | US 2021238309 A1 | 05 August 2021 |
| | | CA 2973964 A1 | 21 July 2016 |
| | | US 2016208018 A1 | 21 July 2016 |
| | | JP 2018503380 A | 08 February 2018 |
| | | JP 6865688 B2 | 28 April 2021 |
| | | WO 2016115559 A1 | 21 July 2016 |
| | | AR 103442 A1 | 10 May 2017 |
| | | KR 20170128234 A | 22 November 2017 |
| WO 2021057822 A1 | 01 April 2021 | US 2022356246 A1 | 10 November 2022 |
| CN 113164621 A | 23 July 2021 | WO 2021115497 A2 | 17 June 2021 |
| | | WO 2021115497 A3 | 21 October 2021 |
| | | TW 202222349 A | 16 June 2022 |
| WO 2020063676 A1 | 02 April 2020 | CA 3114137 A1 | 02 April 2020 |
| | | JP 2022511351 A | 31 January 2022 |
| | | BR 112021004656 A2 | 01 June 2021 |
| | | EP 3858386 A1 | 04 August 2021 |
| | | EP 3858386 A4 | 12 October 2022 |
| | | KR 20210066833 A | 07 June 2021 |
| | | US 2021353764 A1 | 18 November 2021 |
| | | AU 2019349207 A1 | 08 April 2021 |
| | | TW 202027795 A | 01 August 2020 |
| CN 110891972 A | 17 March 2020 | CA 3068200 A1 | 10 January 2019 |
| | | WO 2019008379 A1 | 10 January 2019 |
| | | MX 2019015349 A | 20 July 2020 |
| | | EP 3645565 A1 | 06 May 2020 |
| | | EP 3645565 B1 | 15 December 2021 |
| | | ES 2906361 T3 | 18 April 2022 |
| | | AU 2018295479 A1 | 16 January 2020 |
| | | EP 3985026 A1 | 20 April 2022 |
| | | GB 201710838 D0 | 16 August 2017 |
| | | US 2020157218 A1 | 21 May 2020 |
| | | US 11306142 B2 | 19 April 2022 |
| | | JP 2020529839 A | 15 October 2020 |
| | | US 2022204621 A1 | 30 June 2022 |
| | | DK 3645565 T3 | 14 February 2022 |
| US 2020354448 A1 | 12 November 2020 | GB 201710835 D0 | 16 August 2017 |

Form PCT/ISA/210 (patent family annex) (July 2022)

**INTERNATIONAL SEARCH REPORT**
**Information on patent family members**

International application No.
**PCT/CN2022/142644**

| Patent document cited in search report | Publication date (day/month/year) | Patent family member(s) | | | Publication date (day/month/year) |
|---|---|---|---|---|---|
| | | AU | 2018297630 | A1 | 16 January 2020 |
| | | JP | 2020530272 | A | 22 October 2020 |
| | | WO | 2019008377 | A1 | 10 January 2019 |
| | | US | 11466083 | B2 | 11 October 2022 |
| | | EP | 3649152 | A1 | 13 May 2020 |
| | | MX | 2019015348 | A | 20 July 2020 |
| | | CA | 3068196 | A1 | 10 January 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111623091
- CN 202210230033X
- US 4816567 A
- US 5500362 A
- US 5821337 A
- US 20050238649 A1
- US 5208020 A
- WO 2018237335 A
- WO 2020063676 A


### Non-patent literature cited in the description


- *J. biol. chem,* 1968, vol. 243, 3558
- **KABAT et al.** Sequences of Proteins of Immunological Interest. Public Health Service, National Institutes of Health, 1991
- **MARTIN.** *ACR. Protein Sequence and Structure Analysis of Antibody Variable Domains[J,* 2001
- **LEFRANC, M.P. et al.** *Dev. Comp. Immunol.,* 2003, vol. 27, 55-77
- *Front Immunol.,* 16 October 2018, vol. 9, 2278
- **MARKS et al.** *Bio/Technology,* 1992, vol. 10, 779-783
- **BARBAS et al.** *PNAS,* 1994, vol. 91, 3809-3813
- **SCHIER et al.** *Gene,* 1995, vol. 169, 147-155
- **YELTON et al.** *J.Immunol.,* 1995, vol. 155, 1994-2004
- **JACKSON et al.** *J.Immunol.,* 1995, vol. 154 (7), 3310-9
- **HAWKINS et al.** *J. Mol. Biol.,* 1992, vol. 226, 889-896
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **PRESTA.** *J. Allergy Clin. Immunol.,* 2005, vol. 116, 731
- *Meth. Mol. Biol.,* 2004, vol. 248, 443-463
- *Prot. Sci.,* 2000, vol. 9, 487-496
- **HARLOW ; LANE.** Antibodies. Cold Spring Harbor Press
- **JUNGHANS et al.** *Cancer Res.,* 1990, vol. 50, 1495-1502
- **RAVETCH ; KINET.** *Annu. Rev. Immunol,* 1991, vol. 9, 457-92
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **WATSON et al.** Molecular Biology of the Gene. The Benjamin/Cummings Pub. Co, 1987, 224
- Antibodies: A Laboratory Manual. Cold Spring Harbor Press
- **CHARI et al.** *Cancer Research,* 1992, vol. 52, 127-131
- **SAMBROOK et al.** Molecular Cloning: A Laboratory Manual. Cold Spring Harbor Laboratory Press
- **AUSUBEL et al.** Current Protocols in Molecular Biology. Greene Publishing Association, Wiley Interscience
- *CHEMICAL ABSTRACTS,* 646502-53-6